# EUROPEAN PATENT APPLICATION

(11) **EP 2 993 238 A1**
(43) Date of publication of application: **09.03.2016**
(21) Application number: 14792250.4
(22) Date of filing: 30.04.2014
(51) Int. Cl.: C12Q 1/68

(54) **PROGNOSIS OF RESPONSE TO TREATMENT WITH ANTI-TNF-ALPHA IN PATIENTS WITH RHEUMATOID ARTHRITIS**

(30) Priority: 03.05.2013 ES 201330650
(71) Applicant: Fundació Hospital Universitari Vall d'Hebron - Institut de Recerca, 08035 Barcelona (ES)
(72) Inventor: MARSAL BARRIL, Sara, E-08021 Barcelona (ES); JULIA CANO, Antoni, E-08010 Barcelona (ES); TORNERO MOLINA, Jesus, E-28006 Madrid (ES)
(74) Representative: Campbell, Patrick John Henry
(86) International application number: PCT/ES2014/070377
(87) International publication number: WO 2014/177746

(57) **Abstract**

The present invention refers to the use of SNP rs3794271, and/or a SNP that is in total linkage disequilibrium with this SNP, as a predictive marker of the response to anti-TNF treatment in a patient with RA. The invention also refers to methods to predict the response to treatment with anti-TNF agents, as well as to decide or recommend a treatment for a patient with RA, based on the determination of rs3794271 genotype and/or a SNP that is in total linkage disequilibrium with this SNP.

## Description

The present invention is related to the rheumatology field, more specifically, with the response to tumor necrosis alpha factor inhibitors in patients with rheumatoid arthritis.

### STATE-OF-THE-ART

Rheumatoid arthritis (herein referred as RA) is a systemic autoimmune disease, characterized by the presence of a chronic inflammation mainly in the joints. This chronic inflammation is responsible for the progressive destruction of the joints, leading to different degrees of deformity and/or functional impairment. In certain patients, the disease can also have an extraarticular behavior, affecting different organs or systems like the eyes, lungs, pleura, heart and pericardium, skin or blood vessels. Although RA has a yet unknown cause, autoimmunity is known to play a central role in the disease chronification and development. RA can be a very painful and disabling disease.

Approximately 1 % of the world's population has RA, being up to three times more frequent in women than in men. Although it can appear at any age, it is more prevalent between the fourth and fifth decades of life.

Tumor necrosis factor alpha inhibitors (herein referred as anti-TNF alpha agents), such as etanercept, adalimumab and infliximab, are proving to be highly successful in the treatment of patients with RA. These drugs are able to slow down the progression of the inflammatory process that perpetuates the disease and, therefore, they are able to reduce the disease activity and significantly improve the quality of life of patients.

Despite this improvement in RA treatment, approximately 20 to 40% of RA patients do not respond significantly to anti-TNF alpha agents or they are unable to maintain the initial positive response to these treatments. The group of patients that do not respond to anti-TNF therapy could therefore benefit from alternative drugs with different mechanisms of action like tocilizumab, abatacept or rituximab. It is now well established that an intense treatment at earlier stages of the disease can be crucial for the positive evolution of patients with RA. For this reason, it would be of high clinical value to be able to predict those patients that will not respond to this treatment. An early prognosis of anti-TNF alpha agent response would help to discard said treatments and allow to use an alternative therapy, which would be highly beneficial for patients since the likelihood of a positive treatment response will be increased and the side effects associated to anti-TNF alpha agents will be avoided. Also, from an economic perspective, and given the high costs of RA treatments, the optimization of clinical decision making and treatment selection would be associated to a significant reduction in the associated health care spending.

Previous studies have analyzed the association of several markers with the response to anti-TNF therapy. Some of these include, the polymorphisms in the promoter region of the gene codifying for the TNF alpha cytokine, as well as polymorphisms in the *HLA-DR* gene (major histocompatibility complex, class II, DR), *FCGR3* (Fc receptor gamma III) and *IL1RN* (interleukin 1 receptor antagonist) genes. However, the results obtained from these studies have been so far contradictory. More recently, some genome-wide association studies (GWAS) have found certain polymorphisms associated with the response to anti-TNF alpha (Liu C, et al, "Genome-wide association scan identifies candidate polymorphisms associated with differential response to anti-TNF treatment in rheumatoid arthritis", Mol Med, 2008, vol. 14, pp. 25 575-81; Plant D et al, "Genome-wide association study of genetic predictors of anti- tumor necrosis factor treatment efficacy in rheumatoid arthritis identifies associations with polymorphisms at seven loci", Arthritis Rheum, 2011, vol. 63, pp. 645-53; Krintel SB, et al, "Investigation of single nucleotide polymorphisms and biological pathways associated with response to TNF alpha inhibitors in patients with rheumatoid arthritis", 30 Pharmacogenet Genomics, vol. 22, pp. 577-89). However, none of these polymorphisms has demonstrated a sufficiently reliable predictive ability. Ultimately, there are yet no markers that have sufficient reliability or robustness to be used in the clinical practice.

For all these reasons, it is necessary to identify markers for prediction of response to anti-TNF agents that can be used in the clinical practice and allow to reliably identify those patients with a negative response to anti-TNF therapies.

### DESCRIPTION OF THE INVENTION

The inventors have found that single nucleotide polymorphism (herein referred as SNP) rs3794271 has a high ability to predict the response to anti-TNF alpha therapy in patients with RA. Surprisingly, the research performed by the inventors demonstrate that the association of said SNP with the response to anti-TNF alpha therapy is significant at the genome-wide level. All these experiments are shown in the experimental section of the present document. Based on these results, it can be concluded that SNP rs3794271 is a very powerful marker to predict the response of RA patients to this group of drugs.

Thus, one aspect of the invention refers to the use of SNP rs3794271 as a predictive marker of the response to anti-TNF alpha treatment in a patient with RA.

The examples below show that SNP rs3794271 predicts the response to treatment more accurately when the anti-TNF agent is infliximab or etanercept. Therefore, in a specific embodiment, the invention refers to the use of SNP rs3794271 as a marker in the prediction of the response to treatment with infliximab or etanercept in a patient with RA.

Compared to previous markers described in the state-of-the-art, rs3794271 has shown to have a sufficiently high predictive ability to be used as a tool for decision making in the clinical practice, thereby giving a highly valuable information for the optimization of the pharmacological treatment for each RA patient.

The term "single nucleotide polymorphism" refers to a variation in the DNA sequence that affects a single base (adenine (A), timine (T), cytosine (C) or guanine (G)) of the sequence of the genome. In some instances, however, in the present state-of-the-art, changes in a low number of nucleotides as well as small insertions and deletions (indels), can also be considered as SNPs. If the SNP has a frequency higher than 1% in the general population it is considered a common variant, while it is considered a rare variant if the frequency in the population is lower than 1%. In the present invention, the terms "SNP" and "single nucleotide polymorphism" are used indistinctively, and include both single nucleotide polymorphisms as well as variations that include changes in a few nucleotides, and small deletions or insertions.

SNP rs3794271 is found in the fourth intron of *SLCO1C1* gene in human chromosome region 12p12.2 (NCBI reference sequence of chromosome 12: NC_000012.11, publication date: 31st august 2012) and defines genetic variant in position 20,860,093 of said chromosome (according to GRCh37/hg19 human genome assembly, February 2009).

Given that human beings are diploids (that is, they have two sets of autosomal chromosomes in each cell), any polymorphism will be present in both chromosome, maternal and paternal. Each SNP has two single-base sequences which are called "alleles". In relation to SNP rs3794271, this means that an individual can have the G allele in both chromosomes (i.e. G homozygous individual for this SNP, or GG individual). Alternatively, the individual could have the alternative form A in both chromosome in the SNP location, that is, two A alleles for this SNP (i.e. A homozygous individual, or AA individual), or it could also have one allele of each type (i.e. heterozygous individual, GA or AG).

The inventors have found that, in patients with RA, the presence of the G allele in SNP rs3794271 position indicates a bad response to treatment with either infliximab or etanercept. According to the results of the inventors, heterozygous individuals for this SNP (i.e. one G allele) have a high likelihood of no response to infliximab or etanercept. In the case of GG homozygous patients, this likelihood of no response is even bigger. The genotyping of SNP rs3794271 therefore allows a high accuracy prediction of the response of a patient with RA to the treatment with these drugs.

Thus, another aspect of the present invention refers to an *in vitro* method for the prediction of the response to an anti-TNF alpha treatment in a patient with RA. This method comprises the determination, from a sample obtained from the patient, of rs3794271 SNP genotype, where the presence of, at least, one G allele (in rs3794271 position) is indicative of a bad response to treatment. This same aspect could be formulated as an *in vitro* method to predict the response in a RA patient to anti-TNF alpha treatment which comprises determining, from a sample obtained from the patient, the genotype for rs3794271 SNP, where the presence of two alleles other than G in rs3794271 SNP position, is indicative of a good response to treatment. In other words, the presence of the alternative form A in both alleles, that is the presence of two A alleles in the position of SNP rs3794271, indicates a high likelihood of good response to treatment.

In a specific embodiment, the *in vitro* method of this invention is designed to determine the response to an anti-TNF alpha treatment, selecting between infliximab or etanercept.

In another particular embodiment, the predisposition to a bad response to infliximab or etanercept is associated to the presence of the two G alleles (in rs3794271 SNP position), that is, the predisposition to a bad response is associated to individuals that are GG homozygous in the SNP position.

"Bad response" refers to patients that do not respond to the treatment. "Good response" refers to patients that respond positively to treatment. Treatment response can be determined using known methods in the state-of-the-art, such as the EULAR response (Fransen J et al, "The Disease Activity Score and the EULAR response criteria", Clin Exp Rheumatol, 2005, vol. 23 (Supl. 39)). The EULAR response classifies patients that are under a specific therapy as good, moderate or non-responders. In clinical studies, patients with a good or moderate response have shown to have a significant improvement in the functional capacity and a reduced progression of joint damage, while non-responder patients do not show a significant reduction in disease activity.

The EULAR response criteria use the DAS28 to determine the response category. The DAS28 is score that is obtained after counting the presence of swelling and/or pain in 28 specific joints, and integrating this value with the erythrosedimentation rate (ESR) and, optionally, the patient's global assessment using a visual scale that ranges from 0 to 100. In a continuous scale that goes from 0 to 9.4, the level of activity of the disease according to DAS28 is considered Low (DAS28 ≤ 3.2), Moderate (3.2 < DAS28 ≤ 5.1), or High (DAS28>5.1). The EULAR response classifies patients based on the magnitude of the DAS28 change from the basal point to the endpoint, as well as the activity level at the endpoint. A reduction of 1.2 (2 times the measurement error) in the DAS28 of an individual patient is considered a significant change. This way, a patient that has a significant change (DAS28 reduction > 1.2) and also reaches a low level of activity (endpoint DAS28 ≤ 3.2) is classified as a good responder. If a patient does not reduce the DAS28 in more than 0.6, no matter which is the endpoint DAS28, it is considered a non-responder to therapy. Also, patients that show a DAS28 reduction between 0.6 and 1.2 but their endpoint DAS28 is higher than 5.1, are also considered non-responder patients. The rest of patients, that its, those who do not enter in the EULAR good or EULAR non-responder categories, have an intermediate response and are categorized as moderate responders.

Hence, with regards to the EULAR criteria, the predisposition to a bad response indicates that the patient is an EULAR non-responder, while the predisposition to a good clinical response indicates that the patient is a good or moderate EULAR responder. In a particular embodiment of the present invention, the predisposition to a good responder indicates that the patient is a good EULAR responder.

The method in the present invention provides the medical specialist a very useful tool to decide which is the most adequate pharmacological treatment for a patient with RA. According to the method in this invention, the specialist will be able to discard the treatment with infliximab and etanercept in a specific patient classified as predisposed to a bad response, and recommend an alternative treatment which will have a higher likelihood of response in said patient. The patient will therefore avoid being treated with an ineffective treatment, saving a valuable time for his/her recovery and avoiding side effects associated with the treatment with infliximab or etanercept. Also, according to the method of this invention, those patients that have predisposition to a good response (i.e. those patients that present two different alleles other than G for SNP rs3794271), will benefit from the treatment with infliximab or etanercept.

An aspect of the invention refers to a method to decide or recommend a treatment for a patient with RA which comprises the determination of SNP rs3794271 SNP. If the patient presents, at least, one G allele copy in rs3794271 SNP position, that is if the patient has a bad response predisposition to treatment, a therapy other than anti-TNF alpha agents will be recommended. In a particular embodiment, the anti-TNF agents that will be excluded are infliximab and etanercept. In a particular embodiment, the therapy that excludes infliximab and etanercept is recommended if the patient has the G polymorphic variant in both alleles, that is, if the patient is GG homozygous for SNP rs3794271. In another embodiment, if the patient presents two alleles different than G (generally two A alleles) in rs3794271 SNP position, that is, if the patient has a predisposition to a good response to treatment, a therapy that includes infliximab or etanercept is included.

In a particular embodiment, the method to decide or recommend a treatment for a patient with RA according to the invention comprises, also, the determination of clinical variables of the patient and/or the determination of other biological markers.

It is generally accepted that genetic variations are never isolated in the genome. Given that SNPs are part of a continuous DNA chain (i.e. chromosome), there will be other close variants in the chromosome that will be co-inherited with a very similar frequency and, therefore, will contain a similar level of information to the polymorphism of interest. These close variants that are in high correlation are known as linkage disequilibrium blocks. These blocks, that characterize the whole human genome and have been characterized by the international HapMap project, are clearly defined regions that show a low probability of recombination. SNP rs3794271 is located in a linkage disequilibrium block, defined in SEQ ID NO: 1, and extends from exon 13 of the transcribed sequence of *PDE3A* gene (NCBI reference sequence: NM_001244683, reference sequence NC_000012.11, GRCh37.p10 primary assembly, 30th October 2012) up to the sixth exon of *SLCO1C1* transcribed sequence (NCBI reference sequence: NM_001145945, reference sequence NC_000012.11, GRCh37.p10 primary assembly, 30th October 2012), and including the intergenic region between the two genes. Within this region it is possible to find SNPs that are in very high linkage disequilibrium with rs3794271 SNP, that is, SNPs whose alleles have a very significant correlation with rs3794271 alleles. Linkage disequilibrium is a measure of correlation between the allelic frequencies of two polymorphic genetic loci located in the same chromosome. In most cases, linkage disequilibrium extends up to very short distances, generally in the kilobase range. The region defined by SEQ ID NO:1 constitutes a bad response locus to the treatment with anti-TNF (preferentially, infliximab and etanercept), given that an expert in the area will understand that the SNPs that are in linkage disequilibrium with rs3794271 in this region represent the same response prediction ability than rs3794271.

Therefore, the present invention also contemplates the use of a SNP that is in linkage disequilibrium with rs3794271 as a marker of response prediction to treatment with an anti-TNFalpha agent in a patient with RA. In a particular embodiment, the anti-TNF alpha agent is infliximab or etanercept. Furthermore, the invention contemplates an *in vitro* method to predict the response of a patient with RA to the treatment with an anti-TNF alpha agent which comprises the determination, from a sample obtained from the patient, of the genotype of a SNP that is in linkage disequilibrium with rs3794271, where the presence of, at least, one allele correlated with rs3794271 G allele is indicative of bad response to treatment. This same aspect could be formulated as an *in vitro* method to predict the response of a patient with RA to the treatment with an anti-TNF alpha therapy that comprises the determination, form a sample obtained from the patient, of the genotype of a SNP that is in linkage disequilibrium with rs3794271, where the presence of two alleles non-correlated with allele G of rs3794271 is indicative of good response to treatment. In a particular embodiment, the selected anti-TNF alpha is infliximab or etanercept.

By "correlated allele" we understand the allele (polymorphic form) that is in linkage disequilibrium with allele G (polymorphic form G) at rs3794271 (r² >50%). This means that the G allele at SNP rs3794271 and the allele correlated with it, will be coinherited at a very high probability.

Preferentially, the SNP in linkage disequilibrium according to the invention is found in the locus for predisposition a to a bad response to infliximab or etanercept, defined by SEQ ID NO: 1.

In a particular embodiment of the invention the SNP in linkage disequilibrium with SNP rs3794271 is selected from the group of SNPs defined in Table 3. In another particular embodiment, the SNP in linkage disequilibrium is a SP that is in high linkage disequilibrium with rs3794271. By SNP in "high linkage disequilibrium" we refer to those SNPs whose linkage disequilibrium coefficient in relation to rs3794271 is higher than 0.8 (see Table 3).

The invention also contemplates the combination of SNPs that are in linkage disequilibrium with rs3794271 as markers for the prediction of the response to treatment with anti-TNF alpha, preferentially infliximab or etanercept in a patient with RA, as well as an *in vitro* method to predict the response of a patient with RA to treatment according to the invention that comprises the determination of the genotype in a combination of SNPs that are in linkage disequilibrium with rs3794271. In certain embodiments, rs3794271 is combined with, at least, one SNP in linkage disequilibrium with rs3794271, preferentially a SNP that is located in the locus for predisposition for bad response to infliximab or etanercept defined in SEQ ID NO: 1 and that are reflected in Table 3. In other embodiments the SNPs to combine will be selected from the group consisting of rs10770707, rs1473993, rs10770704, rs10770702, rs959346, rs3751218, rs11045392, rs11045390, rs74406912, rs954866 y rs10770701. In particular embodiments, the combination comprises rs3794271 and rs11045392.

The sample necessary to implement the method of the invention can be any tissue or fluid obtained from the body of the patient, for example, blood, serum, plasma, saliva, urine or hair. In a particular embodiment of the invention the sample is blood or saliva. In another particular embodiment, the sample obtained from the patient is a sample of DNA. However, the method of the present invention does not comprise the sample collection step from the patient.

In a particular embodiment, the patient is a population selected from the group that consists in Caucasian population, Asian population and Centroamerican population.

The state-of-the-art of the technique provides different methods to determine the genotype in a specific region. For example, in a particular embodiment, the determination of the genotype comprises the amplification of the nucleic acid chain region where the SNP is located, for example, through the use of the polymerase chain reaction (PCR). The state-of-the-art comprises methods to design primers to amplify any desired region within the known nucleic acid chain. Primer design can be done automatically using bioinformatic programs that are available for the expert in the area.

In a particular embodiment, the determination of the genotype comprises the sequencing of all or part of the nucleic acid chain of the collected sample. In another embodiment, the genotype determination comprises the hybridisation of the nucleic acid chain of the sample with specific probes. Preferentially, hybridisation will be performed in high specificity conditions.

In another embodiment, the determination of the genotype will be performed using one or more DNA analysis techniques selected form the group consisting of restriction fragment-length polymorphisms (RFLP), random amplification of polymorphic DNA (RAPD), amplified fragment length polymorphisms (AFLPD), all of which are genotyping techniques that are well known for the expert in the area.

The determination of the genotype can require several of the aforementioned techniques. For example, the genotype determination can be performed using amplification and hybridization, or using amplification and sequencing, or using amplification and RAPD. The expert in the area will determine in each case which is the most appropriate technique to determine the genotype.

In another aspect, the present invention refers to the use of methods to determine the genotype for rs3794271, and/or at least one SNP that is linkage disequilibrium with rs3794271 in an *in vitro* method as has been previously described, in order to predict the response of a patient with RA to the treatment with an anti-TNF alpha agent, preferentially, an anti-TNF alpha agent selected between infliximab or etanercept. In a particular embodiment, the means are hybridization probes specific for rs3794271 or for a SNP that is in linkage disequilibrium with rs3794271. In another particular embodiment, the means are primers specific for SEQ ID NO:1 amplification or any region comprised within SEQ ID NO:1. In another particular embodiment, the means are specific primers in order to perform the determination of the genotype using RAPD.

An additional aspect of the invention refers to a kit to predict the response to treatment with an anti-TNF alpha agent in a patient with RA that comprises means to determine the genotype for rs3794271 SNP, and/or at least one SNP that is in linkage disequilibrium with rs3794271, as well as instructions to carry out said determination and for the interpretation of the results, where these instructions for the interpretation of the results indicate that the presence of at least one G allele for SNP rs3794271 and/or the presence of, at least, a correlated allele with allele G of rs3794271 for the SNP in linkage disequilibrium is indicative of bad response to treatment. Alternatively, or together with the previous, the instructions for the interpretation of the results could indicate that the presence of two alleles other than G for SNP rs3794271 and/or the presence of the two alleles non-correlated with allele G of rs3794271 in the SNP in linkage disequilibrium is indicative of good response to treatment. In a particular embodiment of this aspect of the invention, the kit predicts the response to an anti-TNF alpha selected between infliximab or etanercept.

In a particular embodiment, the kit comprises the means for determining the genotype of rs3794271 SNP as well as instructions to perform said determination and for the interpretation of the results, where these instructions for the interpretation of the results indicate that the presence of two G alleles at SNP rs3794271 is indicative of bad response to treatment.

Throughout the description and claims, the word "comprise" and its variants do not pretend to exclude other technical characteristics, additives, components or steps. Also, the word "comprise" includes the case "consists of". For those experts in the area, other objects, advantages and characteristics of the invention will be derived in part from the description and in part from the practice of the invention. The following examples will be provided as an illustration, and are not intended to limit the present invention. Also, the present invention covers all the possible combinations of particular and preferred embodiments herein indicated.

### EXAMPLES

### Patients

A total of 315 RA patients were included in the present study. All patients were collected as part of the Immune-Mediated Inflammatory Disease Consortium (IMIDC). The IMIDC is a network of Spanish researchers working on the genomic basis of immune-mediated inflammatory diseases. RA patients were collected from the outpatients' clinics of the rheumatology departments from 12 Spanish University Hospitals. The selection criteria were as follows: a) fulfillment of the 1987 American College of Rheumatology classification criteria for RA b) >18 years old c) Caucasian European born in Spain d) all four grandparents born in Spain e) >2 years of follow-up since diagnosis f) having received an anti-TNF therapy (infliximab, etanercept or adalimumab) as the first biologic treatment and g) baseline DAS28 score ≥ 3.2.

Informed consent was obtained from all participants and protocols were reviewed and approved by local institutional review boards. The present study was conducted according to the Declaration of Helsinki principles.

### Treatment response definition

The response to anti-TNF alpha treatment was measured using the absolute and relative DAS28 changes, as well as the EULAR response. The endpoint DAS28 was measured at 12 weeks of treatment. The absolute DAS28 change (ΔDAS28) is defined as the change in DAS28 between baseline and endpoint scores (DAS28_{baseline} - DAS28_{week12}); EULAR divides the response to anti-TNF alpha into three categories: good, moderate and non-responder.

### DNA extraction, SNP selection and genotyping

Genomic DNA was isolated from venous blood samples using the Chemagic Magnetic Separation Module I (PerkinElmer). A total of 4 SNPs were selected for genotyping: i) 2 SNPs associated with ΔDAS28: rs10520789 at -727 kb from the 5' end of nuclear receptor subfamily 2, group F, member 2 *(NR2FR2)* gene (chromosome 15q26.2), rs11870477 at -264 kb from the 3' end of mitogen-activated protein kinase kinase 6 *(MAP2K6)* gene (chromosome 17q24.3), ii) 1 SNP associated with relDAS28: rs1539909 at -955 kb from the 3' end of cerebellin 2 precursor *(CBLN2)* gene (chromosome 18q22.3), iii) 1 SNP associated with EULAR response: rs3794271 in the fourth intron of solute carrier organic anion transporter family, member 1C1 *(SLCO1C1)* gene (chromosome 12p12.2).

SNP genotyping was performed using the TaqMan genotyping platform (Life Technologies). The selected Taqman assays were: C__30397748_20 (rs10520789, *NR2FR2),* C__32133788_10 (rs11870477, *MAP2K6*), C__7536165_10 (rs1539909, *CBLN2)* and C__27502188_10 (rs3794271, *SLCO1C1*). Thermal cycle conditions were as follows: 50°C for two minutes and 95°C for 10 minutes, followed by 40 cycles of 92°C for 15 seconds and 60°C for one minute. All PCR and end point fluorescent readings were performed using an ABI PRISM7900 HT sequence detection system (Life Technologies). The genotyping error was estimated by genotyping 10% of the samples in duplicate.

### Statistical analysis

Multivariate linear regression was used to test the association with the absolute change in Disease Activity Score (ΔDAS28) using the baseline DAS28 as a covariate. The allelic X² test was used to test for association with the European League Against Rheumatism (EULAR) extreme response. Bonferroni adjustment of the significance level was performed to account for multiple testing (α = 0.0124). All association analyses were performed using R statistical software (http://cran.r-project.org/) Power calculations were performed using Quanto (v 1.2.4, http://hydra.usc.edu/gxe/) and the online Genetic Power Calculator (http://pngu.mgh.harvard.edu/-purcell/gpc/). All SNPs were tested for deviations from Hardy-Weinberg Equilibrium.

In order to investigate the effect of potential confounders of the observed genetic association with EULAR response, a logistic regression model was fitted. Evaluated variables were age at diagnosis, age at anti-TNF treatment, disease duration, baseline DAS28, smoking habit, gender, number of erosions, presence of Rheumatoid Factor (RF) and presence of anti-cyclic citrullinated peptide antibodies (anti-CCP). The presence of a drug type-specific effect was also evaluated by fitting a logistic regression model including an interaction term between SNP genotype and anti-TNF therapy type as described in Plant et al (supra).

### RESULTS

Using a cohort of 315 RA patients four candidate loci previously identified in a GWAS for anti-TNF response were tested. The call rate for the four SNPs was >99% and the genotyping error was 0%. All four SNPs were in Hardy-Weinberg Equilibrium (P > 0.5). The clinical features of our patient cohort are shown in Table 1.

**Table 1. Clinical characteristics of patients**

| | |
|---|---|
| Age at diagnosis, mean ±SD | 43 ± 12 |
| No. (%) female | 257(81) |
| Disease duration (years), mean ±SD | 11 ± 8 |
| No. (%) Anti-CCP positive* | 239(79) |
| No. (%) RF positive* | 246(78) |
| Baseline DAS28, mean ±SD | 5.5 ± 1.1 |
| No. (%) treated with infliximab* | 115(36) |
| No. (%) treated with etanercept* | 113(36) |
| No. (%) treated with adalimumab | 87(28) |
| Smoking habit^{a} | 78(25) |
| No. (%) EULAR None Responders^{b} | 77(24) |
| No. (%) EULAR Good Responders^{b} | 105(33) |

| | |
|---|---|
| ^{a}Current smokers. ^{b}Determined at week 14 | |

In the EULAR good vs. none response a highly statistically significant association between *PDE3A-SLCO1C1* SNP rs3794271 with treatment response was found (P = 1.74E-5, OR (95% CI) = 2.63 (1.68-4.12). Furthermore, adding the EULAR moderate group of anti-TNF responders to the good responder group, the association was still highly significant (P = 2.69E-4, OR (95%CI) = 1.98 (1.37-2.89)). None of the other tested markers showed significant association even at the nominal level (P < 0.05). rs3794271 genotype frequencies for all three EULAR categories and average ΔDAS28 and relDAS28 scores are shown in Table 2.

**Table 2. Genotypic frequencies of rs3794271 for the three EULAR categories and the average ΔDAS values.**

| **rs3794271 genotype** | AA | | AG | | GG | |
|---|---|---|---|---|---|---|
| **EULAR response** | Number (%) | ΔDAS (mean ± SD) | Number (%) | ΔDAS (mean ± SD) | Number (%) | ΔDAS (mean ± SD) |
| EULAR GOOD | 58(18.5) | 2.77 ± 1.0 | 41 (13.1) | 2.7 ± 1.0 | 5 (1.6) | 2.4 ± 0.6 |
| EULAR MODERATE | 53 (16.9) | 1.7 ± 0.8 | 67 (21.4) | 1.6 ± 0.7 | 12 (6.4) | 1.9 ± 1.0 |
| EULAR NONE | 20(6.4) | 0.0 ± 1.1 | 43 (13.7) | 0.1 ± 0.7 | 14 (4.5) | -0.2 ± 1.0 |

The results demonstrate that SNP rs3794271 is associated with the prediction of the response to anti-TNF alpha therapy. The ability of the SNP to predict those patients that are bad responders according to EULAR criteria (compared to good responders) is very high, and also to predict those patients that are bad responders according to the EULAR criteria (differentiating them from good and moderate).

In a secondary analysis, it was evaluated the observed genetic association after controlling for potential confounders. For any clinical variable analyzed as a potential confounder, the change in the SNP regression coefficient and statistical significance was negligible. This result confirms that the relation between the SP and the anti-TNF response is stable and independent of any clinical variable. Also, the presence of an interaction with each of the clinical covariates was tested by adding an additional interaction term to the logistic regression model, but none was found to be significant. This result confirms that the SNP association does not interact with any other clinical variable. However, testing for drug type-specific effects a significant interaction between the presence of adalimumab and anti-TNF response was found (*P* = 0.045). Using the Breslow-Day method to test for differences in the association between adalimumab treated patients against the other two treatments a suggestive evidence of heterogeneity was found (*P* = 0.078). Testing each treatment separately, no significant association of SNP rs3794271 with response in adalimumab-treated patients was found (*P* = 0.37), while there was substantial evidence of association for infliximab (*P* = 0.0046) and etanercept (*P* = 0.00028). These results indicate that the ability of SNP rs3794271 to predict the response to anti-TNF therapy is more accurate when it refers to anti-TNF drugs infliximab and etanercept.

Additionally, a meta-analysis was performed to combine the association evidence between SNP rs3794271 with the response to anti-TNF alpha therapy from the present study with that from the previous GWAS performed by Krintel et al. (supra). The fixed-effects model implemented in the "rmeta" R package was used to estimate the summary Odds Ratio (OR). The METAL software tool (http://www.sph.umich.edu/csg/abecasis/metal/index.html) to calculate the combined P-value. In this approach, the significance values are weighted according to the sample size of each study. ORs were calculated according to the minor allele and to the risk of being EULAR none responder. Variance in liability explained by *PDE3A-SLCO1C1* SNP was estimated in the replication sample using the approach proposed by So et al ("Evaluating the heritability explained by known susceptibility variants: a survey of ten complex diseases", Genet Epidemiol, 2011, vol. 35, pp. 310-7). Combining the statistical evidence from the GWAS and the present association study, a genome-wide significant association of PDE3A-SLO1C1 SNP rs3794271 with anti-TNF treatment response was found (P = 3.34E-10). The estimated summary effect size for this genetic association was OR(95% CI) = 2.91 (2.57-3.25).

We evaluated the allele dose effect by comparing the effect size with one or two copies of the minor allele (G). The effect size for the heterozygote genotype was OR(95% CI) = 3.0 (1.6-5.9). The effect size for the homozygote was even bigger OR(95% CI) = 8.1 (3.3-19.7). These results indicate that the association between a bad response to anti-TNF alpha treatment for heterozygote individuals at the rs3794271 SNP position is very high, and that this association is even bigger for GG homozygous individuals.

A study of the genomic region that harbors rs3794271 was also performed. This study revealed the existence of two peaks of high recombination levels ("hotspot" regions) in the 13th exon of the transcribed sequence of *PDE3A* gene and in the sixth exon of the transcribed sequence of gene *SLCO1C1,* which delimit a linkage disequilibrium block that is clearly differentiated from the rest of the genome. This block, defined by SEQ ID NO: 1, concentrates SNPs that are in high linkage disequilibrium with rs3794271. These results indicate that the linkage disequilibrium block defined by SEQ ID NO: 1 constitutes a locus that predisposes to the response to anti-TNF alpha treatment. The sequence corresponding to the *PDE3A* gene that is included in this predisposing locus goes from position 44,910 from sequence number 1 to position 61,065. SEQ ID NO: 1 contains also the intergenic sequence between *PDE3A* and *SLCO1C1* (from position 33,663 to position 44,909). SNP rs3794271 is located in position 56,714 from SEQ ID NO: 1.

Table 3 includes those SNPs from the sequence that predisposes to a bad response to treatment (SEQ ID NO: 1) and that are in linkage disequilibrium with rs3794271, as well as the minor allele (A1), the major allele (A2) and the allele correlated with variant G (risk allele) for each of them. The risk allele is the polymorphic form in the location of each of the SNPs that is in linkage disequilibrium with the G allele for of SNP rs3794271. Risk alleles, therefore, yield the same information with regards to the predisposition to treatment response.

**Table 3. SNPs in linkage disequilibrium with SNP rs3794271 in the locus for the predisposition to bad response to infliximab or etanercept treatment.**

| Position in SEQ ID1 NO: 1 | Reference | Position in chromosome 12 | LD¹ | A¹ | A² | Risk Allele^{2, 3} |
|---|---|---|---|---|---|---|
| 7739 | rs4451779 | 20811118 | 0.60 | T | G | T |
| 13643 | rs10743388 | 20817022 | 0.66 | C | G | C |
| 14099 | rs10770689 | 20817478 | 0.60 | T | C | T |
| 15772 | rs6487129 | 20819151 | 0.58 | G | A | G |
| 17061 | rs10770691 | 20820440 | 0.58 | C | G | C |
| 17492 | rs11045376 | 20820871 | 0.56 | C | T | C |
| 18105 | rs11045378 | 20821484 | 0.57 | T | A | T |
| 24396 | rs10770695 | 20827775 | 0.63 | T | C | T |
| 25316 | rs12301364 | 20828695 | 0.61 | A | G | A |
| 26141 | rs10841593 | 20829520 | 0.61 | G | A | G |
| 27013 | rs151131008 | 20830392 | 0.61 | TTTTTC | T | TTTTTC |
| 28335 | rs11045385 | 20831714 | 0.62 | T | C | T |
| 29216 | rs10770701 | 20832595 | 0.81 | A | G | A |
| 30640 | rs954866 | 20834019 | 0.85 | A | G | A |
| 34206 | rs3809209 | 20837585 | 0.74 | C | T | C |
| 34785 | rs7305718 | 20838164 | 0.77 | C | T | C |
| 37058 | rs74406912 | 20840437 | 0.85 | GT | G | GT |
| 37436 | rs11045390 | 20840815 | 0.85 | T | C | T |
| 37460 | rs11045392 | 20840839 | 0.84 | T | C | T |
| 37742 | rs2203493 | 20841121 | 0.75 | A | T | A |
| 38709 | rs137927950 | 20842088 | 0.66 | T | TTC | T |
| 40201 | rs2417861 | 20843580 | 0.77 | C | T | C |
| 45688 | rs3751218 | 20849067 | 0.95 | G | A | G |
| 48707 | rs959346 | 20852086 | 0.83 | G | A | G |
| 48942 | rs10770702 | 20852321 | 0.83 | T | G | T |
| 50162 | rs10743389 | 20853541 | 0.51 | T | C | T |
| 52382 | rs10770704 | 20855761 | 0.83 | T | C | T |
| 52973 | rs71939085 | 20856352 | 0.59 | TTGA | T | TTGA |
| 53200 | rs10743390 | 20856579 | 0.58 | T | C | T |
| 53894 | rs11392906 | 20857273 | 0.69 | T | TC | T |
| 53895 | rs201855778 | 20857274 | 0.69 | G | GC | G |
| 54088 | rs10770705 | 20857467 | 0.53 | A | C | A |
| 54097 | rs10770706 | 20857476 | 0.69 | G | A | G |
| 54283 | rs78690605 | 20857662 | 0.69 | C | CAGAT | C |
| 54906 | rs10505868 | 20858285 | 0.69 | C | T | C |
| 56271 | rs1473993 | 20859650 | 0.87 | C | T | C |
| 56605 | rs3838816 | 20859984 | 0.54 | AT | A | AT |
| 56714 | rs3794271 | 20860093 | 1.00 | G | A | G |
| 58072 | rs10770707 | 20861451 | 0.99 | A | T | A |

| | | | | | | |
|---|---|---|---|---|---|---|
| ¹LD = linkage disequilibrium coefficient ²More than 1 nucleotide means that the SNP is an insertion/deletion ³Calculated in Caucasian population | | | | | | |

In summary, strong evidence was found for the association between the locus defined by genes *PDE3A* and *SLOC1C1,* and their intergenic region, particularly the region defined by SEQ ID NO: 1 with the response to anti-TNF alpha treatment in patients with RA. Based on the obtained results, this association is particularly significant for infliximab and etanercept drugs and for GG homozygous patients at position rs3794271.

SLCO1C1 is a cell membrane protein that belongs to the OATP1 organic anion transporting polypeptide family. This group of proteins have a wide substrate specificity and been shown to play an important role in the absorption, distribution and excretion of drugs, including methotrexate. To date, the protein encoded by *SLCO1C1* has been mainly associated to thyroid hormone and estradiol transport. However, the fact that this gene encodes for four different transcript variants increases the probability that more functionality will be identified.

*PDE3A* codes for a phosphodiesterase (PDE) that hydrolyzes important secondary messengers like cAMP and cGMP. Cyclic nucleotide PDEs have shown important immunomodulatory functions and they have been considered themselves as important targets for the management of autoimmune diseases including RA. For example, PDE inhibition has shown to reduce TNF production in Lipoplysaccharide stimulated monocytes.

Page 1 of 33

SEQUENCE LISTING

<110> FUNDACIÓ HOSPITAL UNIVERSITARI VALL D'HEBRON-INSTITUT DE
      RECERCA

<120> Anti-TNF alpha treatment response prediction in patients
      diagnosed with rheumatoid arthritis

<130> P2678PC00

<150> P ES201330650
<151> 2013-05-03

<160> 1

<170> PatentIn version 3.5

<210> 1
<211> 61065
<212> DNA
<213> Homo sapiens

<400> 1
taaatgatga tgttggaata gattggacca atgaaaatga tcgtctactg gtttgtcaaa      60

tgtgtataaa gttggctgat atcaatggtc cagctaaatg taaagaactc catcttcagt     120

ggacagatgg tattgtcaat gaattttatg aacaggtaac tgaccactgt ttaatacagc     180

ttaatctgta cttacaggtt gctcatgaat tgctcaaagc ttctaacagc tatgaaagta     240

tgatcactct attttatatt catacttttg tcagaaaatg cactggtcaa ttttgatcag     300

gagacaaaaa gtgacgtata aaaattggac taaaatataa tcatagaact ctatgataaa     360

agttatttgc aagcatttgg gggaagaaga tattgagata gtggaagtca gatgttagga     420

agatccttga aatactgttt ttttatagat ttatttatta tcatagaagg tagtttaaat     480

gtatatggag tagcttgtat gtatatggta gtttaaatgt atatgcatat cacattaatt     540

tcacttactt tcctaaaaaa tttatttatt atcatagaag gtagtttaaa tgtacatgga     600

atagtttata tgtatatgta tatggtagtt cgaatgtatc tgtatattag attaacttca     660

cttttgtaaa aaaattaata agagcattat gtgaaaaagc taaaaagaaa tagacatttc     720

atttgacatg ataactaaga gttaacagac tactcagaaa gctaccacaa ttacataagc     780

tgggggtgat cctcctgaca ttaccctaca cccatcagaa tgcacttgaa ccttcctgtt     840

tggcactgtg aaccactctt aaaaagaatg tggaggtgaa ggagcatgca taaaggacag     900

gaataagtga cagaaaagca tgtggaaaaa aatgaaggta tttactcttg gggaaatata     960

actgtcttca gatatttaaa agagtgacat actaatttag aaatcggtta atcaacattg    1020

ctgtggagat taaagaagat aaattatata acatacttag cacaggatct gattcataaa    1080

aaaagttaga tccctctcta ctgccctaaa aaaggtatga atataccagt ctatagaagt    1140

tattgggagg tagtgttcag gtcaatgtca gaaagaaatt tatattgcta aaggtatcca    1200

aaaaataggc tgagacacca tattaatggt aataaattga ctgtcattgg acatgtttaa 1260

acaagagatt agaatgaaat tttatggtta ctataggtag gattcatata tagaatgaaa 1320

gatttttttt attttattat actttaagtt ttagggtaca tgtgcacaac gtgcaggttt 1380

gttacatata tatacatgtg ccctgttggt gtgctgcgcc cattaactca tcatttagca 1440

ttagatatat ctcctaatgc tatccctccc tcctcccccc accccacaac agtccccagt 1500

gtgtgatgtt ccccttcctc tgtccatgtg ttctcattgt tcagttccca cctatgagtg 1560

agaacatgcg gtgtttggtt ttttgtcctt gcgatagttt gctgagaatg atggtttcca 1620

gtttcatcca tgtccctaca aaggacatga actcatcctt ttttatggct gcatagtatt 1680

ccatggtgta tatgtgccac attttcttaa tccagtctat cattgttgga catttgggtt 1740

ggttccaagt ctttgctatt gtgaatagtg cctcaataaa catacatgtg catgtgtctt 1800

tatagcagca tgatttgtag tcctttgggt atatacccag taatgggatg gctgggtcaa 1860

atggtatttc tagttctaga tccctgagga atagccacac tgacttccac agttgttgaa 1920

ctagtttaca gtcccaccaa cagtgtaaaa gtgttcctat ttctccacat cctctccagc 1980

acctgttgtt tcctgacttt ttaatgattg ccattctaac tggtgtgaga tggtatctcg 2040

ttgtggtttt gatttgcatt tcactgatgg ccagtgatga tgagcatttt ttcatgtgtc 2100

ttttggctgc ataaatgtct tcttttgaga agtgtctgtt catatccttc acccactttt 2160

tgatggggtt gtttgttttt ttcttgtaaa tttgtttgag ttcattgtag attctggata 2220

ttagcccttt gtcagatgag taggttgcaa aaattttctc ctaaaaccat aaaaacccta 2280

gaagaaaacc taggcaatac cattcaggac ataggcatgg gcaaggactt catgtctaaa 2340

acaccaaaag caatggcaac aaaaaccaaa attgacaaat ggggatctca tcaaactaaa 2400

gagcttctgc acagcaaaag aaactaccat cagagtgaac aggcaaccta cagaatggga 2460

gaaagttttg tgtttttaaa acatcatgaa atcatgtatt gcctttccta agaatgggat 2520

taattcataa atctcgaaat atggaaatgc agaaaaatgt aaattaaagg gaaaattcat 2580

gtataataat accacttctt aggcataatt gttgctgata ttttgggata tttcctttat 2640

taattttgct agataactgc tggttaaaaa tctccctttt ttcctcagaa acatttgaag 2700

cacttcaaaa taaaacattt gtacagtaat attattaaac atatagataa ataagaatat 2760

cagaaaccac acaatcacag gagatatatt aaaattcaaa caaaatctgg gataaggtaa 2820

ccccttgaat tgagcaataa attctttaaa gctttctgga agccaaagca aaaaaaggaa 2880

aataggctac atttatatat ttttaaaaaa attgttctca ctattgcatt tttttttttg 2940

agatggagtc tcactctgtc accctggaat gcagtggcac gatctcagct caccgcaacc 3000

tccgcctcct gggttcaagt gattctcctg cctcagcctc ccaagtagct gggattacag 3060

gcacctgcca ctacacccag ctaatttttt gtatttttaa tagagacgga gtttcaccat 3120

gttggccagg ctggtctcga tttgcccact ttggcatccc aaagtgctgg gattacaggc 3180

gtgagccacc acgcccagcc tcaccattgc attttcgtgc atttatattc tcatataaaa 3240

gacactgact tgtgtgatta aaatatcatc tacttataca ttagaattta gactttctgt 3300

atttctcctt gataaaaagc aaaaacctca agttcagaaa agtactaatt agcaacagtg 3360

tttctatagg aaccttagca cttgaggtag gatcctttag ctgtcaagta aagaagtttg 3420

tgcattaatg cctgggtttt tacatattta caaatttcag atgccaggtg aatgttgact 3480

tcacttgtat tttatttcta gggtgatgaa gaggccagcc ttggattacc cataagcccc 3540

ttcatggatc gttctgctcc tcagctggcc aaccttcagg aatccttcat ctctcacatt 3600

gtggggcctc tgtgcaactc ctatgattca gcaggactaa tgcctggaaa atgggtggaa 3660

gacagcgatg agtcaggaga tactgatgac ccagaagaag aggaggaaga agcaccagca 3720

ccaaatgaag aggaaacctg tgaaaataat gaatctccaa gtaagttcta aaacctagtt 3780

ctaatgtgtt ttccctggga ttgctttctt actttagatg aattttatgc ttcttatgaa 3840

atacacaata cttcaagtct aaacatcatt tgcggagttt gactacggat agctcccttc 3900

cccaatatca taataataga aatcccagac tatggaatct ataataataa cacatatgat 3960

acaaagatat cttctccgtc atgaagtcat gaagttagaa aaaaataata ataaggagtt 4020

gaagcctatt cttcagggta tctggttttt tgtttgtttt ttgttttttg aggcagagtc 4080

ttgctctgtc acccaggctg gcgtgcagtg gcacgatctc ggctcactgc aagctccgcc 4140

tcccaggttc atgccattct cctgcctcag cctcctgagt agctgggact acaggtgcgc 4200

aacactacac ccggcttttt tttttttttt tttttttttt gtattttgag tagagatcgg 4260

gtttcaccat gttagccagg atggtctcga tctcctgacc ttgtgatctg cctgcctcga 4320

cctcccaaag tgctgggatt acaggcgtga gccaccgcgc ccagctgctt caggttatgt 4380

tagcgattta aaagtgaaag aatttcagtt accataaaga ccagtcatag gtttttattg 4440

gcttggttag gggaactcca tgtgaataga taggaggtag tattcatttg ttcattcaac 4500

agacatttat tgagcatctg ctctgtgcca ggtatccttc caggtactag agatagagtt 4560

gtggggaaaa aaagacaaaa aataaaataa aatccttgcc cttatagaag tacattctag 4620

tgggatcagt agacaataag caaaacaagt gagtatatct atagtatgtt aaatacagta 4680

ttatttactg tagtgataaa tgctgagaaa tatgagagta aaaataaaag gagggagata 4740

tgaaatatta tagagatagg ttaaaatttt aggtaagata tccagggata ggaaagatgt 4800

aaagcaggga aagatttgta aaaagaaaaa gaaaagacct gcaagaagca agggagtgct 4860

ccctggggct gtatgtggaa agaggattcc aaagagagga gaaagcaacc tgtgcaaagg 4920

tccggggatg tacattgggt ggggtgttca gggagcagca ggagaactgg gatagctgga 4980

gcagcaagac agaggcagcc gggaacgaac tgtccaaggg agagaataca gtcatgggtt 5040

cttagttgct gtttctggtt gggccagtaa agccccttcc tcatcccact gttctgctta 5100

tcactagaga cagaaactaa aaaccatggt ttcaggtgct aaaagcctaa aacaaaacaa 5160

aacagaagaa cagcaacaac aaataagtca gcctggacaa gcttggaagg tgggactctg 5220

taggtcacgg taagtgttgt ggcttttact ctgagtaaga tgggaaatcg tcagtggttt 5280

tgaacggaag agttagtaga ctcaacttac attttaactg gactgcttca gctgctctat 5340

tgagaatatg cttggggttg agaagggagc aaggatagac atagggcggt cagttaggag 5400

accattgaaa ttatccaagt gagagatgcc tgtgactttg aaccagggca gtagcaacag 5460

tgacagtgac aacctatcac atgtgataat tggctggagt gcatcatgtg gccaatctaa 5520

cctatttagc ttaattgact atgaaatcat ttttatttat gcctctaaag ctactctcct 5580

atttatcctg tcatgaaaca aatagtgact aatagcataa ttagtaaaaa tccatgaccc 5640

ttgcaatgat attgcacaaa tactgctcct aagaacatag tatgtgattt ctgtctacct 5700

ttacagataa agtacaatct taatatcctc aaggttctta ccaaaaatac aaccagtgaa 5760

agacattctg atcatttcgt gcctattttt atcacagtca ggataatgca caacttggtt 5820

cattccaacc atcttgtaag gagattaacc ttctactgat ccattttgct ctcatataag 5880

caccttgttg taggcacaca taagcaaaca taaagaagtc cttatcaatt atgaacttat 5940

gaagtaaaat tttgcaaatt gagttttaga tcagatctgg cctgttaatt actttttgta 6000

gaatggggga aaattaaagt tgtttttctt caatatgcta ctgtcaaagt aattatattt 6060

catttatctg atgcaaataa aaagacttcc attagaaaat ttgtactaat cttgagattc 6120

agagctagaa gccaagactt ggaattcatg ggttattcat tcattttttt cacttaataa 6180

acatgtatta gttttctctt tttgtttgtt ttcatttcaa agagccaggt agtttgctgt 6240

cttactaaga ataagacagt ttttccactg aatgaaggta ttgtcagctt agcctctgga 6300

aaagctttgt tcccaggggc cctgacttac tgctctttga actcaaacac atggaaaata 6360

cagacgcaca gatccaggca ttgggaattg aaggacccag gccaggagac ttaacaggct 6420

caacttgtca acctagcctt tagcagcaaa tgctacaacc acatattcca tttctttctg 6480

ttctgtaggg ttgggttgtc ataccttcca gtttttatca agttcactaa agcaggaatg 6540

agagactttt tgggttctgt ttgctcatgc cttccaaaag ttaactgacc acattatatt 6600

acaatttaaa aatatcccat ccacaatgat ttgatactgt agctttggaa atattatctt 6660

taaagtattt ctttacaacc aagaaaatga taaatatgga gcaacattag tatttaccaa 6720

atattggcca catagagtaa cagaatttta gaaatgaagg gggaatcaat gcttatcagt 6780

tagctgcctc actttacata tattaaacta aggctcagaa atttaaatga cattgcaagg 6840

ttgtagaacc agaaagggaa cctaggtctc ttaattttta acttcaatgc ttttggctct 6900

cagtaatgtt aatatcagaa ttcatactgg ctaaaatggt taccttgact ttgcctaata 6960

cttaggtttc agaaaaaaat aaatatgcac agataacttc tgtagtttgt aaaacttgga 7020

gatgtcccag gaaacagaat aaaatgtttc aatatggtat cctaggaaaa gaaggttttt 7080

atcccacctt ataattaaat ctgaaagatt cctacagtga actaagaggt tcctaatgaa 7140

gtactaagct gcatttagaa ttggagcaat gaaaggctct ttgttctcct ttcaaaaggt 7200

tccagtacag aacaatttgg ctcccaatac aggcatgttt tagaccacct gcaggctcta 7260

atctacgcaa atttatttgg tacaaacttt cttctacgtc accttcttat tcagtataat 7320

tccagagagg tttattgcta acatttaaca gtattaataa gagaacagtt gctctcatgg 7380

ggactgccct ggaaggaaaa aagaggaaaa ttgagctttt gttagcttct tccctatgaa 7440

tgcactgcca tcaatgtgta taactttaag atgaaataat caccttaaac tgaagagtgt 7500

ttgctacaac tgtgattcaa acctgtcctt cagacagggg tcctctgtaa ctctcttgat 7560

tcaaaggatt agcttaaaaa gtatattaac atttaaacat ttctcaacag aatttgattt 7620

ataacaaaaa cgaagtagaa atagacaagt agagatagaa gagtaatttt taatttgctt 7680

taaaaaatct gatgtattaa tgtattttat tgactttcat agaaagaatt caaatttatt 7740

taaatttcaa ttgtatctac tgcccttgtt aggaggcttt caaatggaag aattgatata 7800

aggggtcttg gcctgtgccc catacacttt tgcctatgtc ccagttcttc aaactttgct 7860

tggaatactg ctagctcctt ttcacccagt gttatttaat tctcattact tacatccagc 7920

ccaggtacca ggtcctttaa aaaaaaaatt ctacctaatt ttccacagtt tgtgtcattt 7980

gtgttgccat ggtttgatgt atcaaagaga aattgcacca gacatttgtt aaaaatggca 8040

acgaaggcgt tattcaagac tactgtaata ggggagacag attgaactct gttgaagcaa 8100

aaggtgggag aattgttcaa tgctgctgtg agctagtgaa aaagtgctgg aggatgttat 8160

gtgagcattg gttagtatga ttaagccacc tgttaattgg tacttactaa agttaggctc 8220

ctacctccca acagagacta ggagataggc ctgcctgctg gcctaccttt cttctttcct 8280

tccatccttc cttccttcat ttaaatcttt tttaaatttt aaaatttaaa ttttattttg 8340

taagactgca aagctaatta gaggctggga gaagatttat ctatattcca gaggggcaga 8400

gaaagaattt gctattacag gttttctaaa gtaaatgttc taacaagagg gaggtcaggg 8460

gcctatagtc tagaagtcta aaatagtgtc aaactgagag aaatggcaag cccatcttgg 8520

tcacaagaat atgtctgtca taaaacatag cacatgtatt tcagtttttg tcttcttggc 8580

actctctctt acagagagac tgtaaactcc tttaaggcaa gatctgagct taattgatct 8640

gtgtttccaa agtatcctgg aaagtatttg ccagttagtt tgagtccatc tcatgaagaa 8700

attatgaata tcataatttt tttttccttt tgactctgcc tgtgttcatt tccctcagca 8760

Page 9 of 33
ctcattcaat ttaataattg tcattctgtg tctatatggt tcaaaattaa ttcaagataa 8820
tttaatgtcc tttttattca ctgtgtttta aaaattaatt ttattctgta cagtacaaga 8880
aatttctgca gtatatcaaa ataaaactgt taaaagaatg actagccctt ccacaaagat 8940
gttggcagat gacctccaag tttctttgtc acccccatag aaattggctt tttttctttc 9000
attattgtgc tattattaag acttttcttt tttttcttgc tcccagagta agtcatatct 9060
acttcattaa atagacccaa atatcccttt tgatcttcct gctaacttgt actgtttttt 9120
ttaattgttt atttactttt gtagagacag cagtctcact ttatttattt ctgtagaaac 9180
aggacctcac tatgtttccc aggctggttt ggaacccctg acgtcaagtg atcctcttgc 9240
ttcagcctcc caagtagcta agattacagg cagcatgagc caccatagcc agcatgaact 9300
attaactctt taatgcagtc tcaattagat cagcatctcc tgtagaccac tttttcgtat 9360
tagttttatt tttaccactt atcaatactt catcatttac ctggaaatgt ggaacaaaaa 9420
ctagagagaa aattattcat ttagtaaact tttactgaat gctttagtaa agtctaggtc 9480
ttcacagata ttacatatac acaaaagtaa agagaaaagt ccatatacca ggtgatatgg 9540
tttggctgtg ttcccaccca accctcatct tgaattgtag ttcccataat ccccatgtgt 9600
cgtgggaggg acctggtggg aggtaatcga atcataaggg gagttacccc catgctactg 9660
ttctcatgac agtgagtgac ttgtcacaag atttgctggt tttataagag gcttttcccc 9720
ctttccttgg cacttctctc tttgtatgac catgtgaaga aggatgtttg cttccccttc 9780
caccatgatt ataatttcct gaggcctcca tagccctgtc agtcaattaa atctcttttc 9840
tttgtaaatt actcagtctt gggagttctt tatagcagtg tgaaaatgga ctaatacagt 9900
aagttggtac cagaagtggg gtgctgatat aaagataccc aaaattgtgg aagcaacttt 9960
ggaacacagt aacaggcaga gattggaaca gtttggaggg ctcagaagaa gacaggaaaa 10020
tgacagaaag tttggaactt cctggagatt ggagggtcat atggtttggc tgtgtctcca 10080
cccaaatcta atcttaaatt cccatgtgtt gtggagggac ccggtgggag ataattgtat 10140
catcatggca agtcttttct atgctgttct cgtgatagtg aataagtctc acaagatcta 10200
atggttttaa aaagaggagt tcctttgcac aagttctctt tttggctgct gccatccatg 10260
taagatgtga gttgcttctc cttgccttcc tgtatgattg tgaggcttcc tgagcaacat 10320
ggaactgtga gtgagtgctc aattaaacct ctttcctttg taaattgccc agtctcaggt 10380
atgtctttgt cagctgcatg gaaatggacg aatacagtaa attggtactg gtagagttgg 10440
gtgctgctga aaagacaacc aagaatgtgg aaacgacttt ggaactgggt aacaggaaga 10500
ggttggaaca gtttggaggg ctcagaagaa aaaagaaaat gtgagaaagt tagtttggaa 10560
ttttctagag acttgttgaa tggctttgac aaaaaatgct gatagtgata tgaacaatga 10620
agtccaggct gaggtggtct cagatggaga tgaggaactt gtcgagaact ggagcaaatg 10680

Page 7 of 33

tgactcttgt tctgttttag caaagagact ggcagcatta tccccctgcc ctagagattt 10740

gtggaacttt gaacttcaga gagatgattt acggtatctg gtgggagaaa tttctaagca 10800

gcaaagcatt caaaggcaac ttgggtgctc ttaaaggcat tccattttat aagggaagca 10860

gaacataaaa attcagaaaa tttgcagtct ggcaatgcaa tagaaaagaa aatcccattt 10920

tgtgaggata aatccaagcg agctgcagaa atttgcataa gtaatgagga gccaactgtt 10980

aatccccaag acaatgggga aaatgtctcc agtatatgtc agaggtcttc atggcagccc 11040

ctcccatcac aggcctaggg gcctaggagg aaaaagtggt ttaatgggct gggttcaggg 11100

tccctgagca gtgtgcagcc tagggacttg gttccctgca tcccaaccac tccagttgtc 11160

actgaaagag gccaaggtac agcttgggct gttgcttgag agggtggacg cctcaagcct 11220

tggcaccttc catgtggtgt tgagcctgtg agtaaacaga agtaaagaac tgaggtttgg 11280

gaacctctgc ctatatttca gagggtgtac ggaaatgctt ggatgatcag gcagaagttt 11340

gctgcagggg tggggcagtc atgcagaacc tctgctaggg cagtgtggaa gggaaatgta 11400

gggtcagagc taccacacag agttactact ggggcagtgc ctaatggaac tgtgagaaga 11460

gagccactgt cctccagacc ccagaatggt agatccacca acagctggca ccatgtgcct 11520

ggaaaagcca cagacactca atgccagccc atgaaagcat ccatgaggga ggctgtaccc 11580

tgtaaagcca caggggcaga gctgcccaac actataggat gcccctcttt gccttccacc 11640

atgcttgtca ggtctctcca gccatgtgaa actgtaagtc caataaactt ctttcttttg 11700

taaattgccc ggtcttgggt atgtctttat cagtagcatg aaaacggact aatatggagg 11760

actcagaaga caggaaaata taggaaagtt tggaacttct agagacttgt tgaatggcac 11820

tgaccaaaat gctgatagtg atatggacaa taaactccag gctgaggtgg tctcagataa 11880

agatgaggaa cttgttgaga actggagtaa aagtcactct tgttttgctt accctgcaga 11940

gccacagggg caaagctgcc caagaacacc tcctgcatca ttttgacctg gatgtgagac 12000

atggagtcaa aggagatcag ttttgaactt taaggtttaa tgactgccct attgaatttt 12060

agacttgcat ggggcctgta gcgcctttgt ttcagcaagg aaactggtgg cattttgctc 12120

tagccctaga gatctgtgaa actttgaact tgagggagat gatttagggt atctggccga 12180

agaaatctct aagcagcaaa gcactcaaga ggtgactttg gtgctattaa aatctctcag 12240

ttttatgtat tcagaaagac atggtttgga attggaactt atctttaaga gggaagctga 12300

gcataaaaat tcagaaaatt tgcaacctga ggatgtgata gaaaagaaaa acccattttt 12360

tgaggagaaa ctcaagtggg ctgcagaaaa tttgcataag taacaaggag ccaaatgtta 12420

atcaccaaga caatggagaa aatgtctcca gggcatgtca gagaccttag tggcagtccc 12480

tcctatcata gacccagagg cctaggcaga aaaaacgatt tcacaggcca ggccccagac 12540

cctctgctgt gtgcagccta gggacttggt gccgtgagtc ccagctgttt cagccatggc 12600

agaaaggggc caatgtacag ctcaggttgt ggcttcagag ggtgcaagcc cgaagccttg 12660

gcaacttcca tatcatgtta ggtctgtggt gtgcagaaga cagaaattga ggtctgggaa 12720

cctccaccta gattaaagag gatgtatggc aatgcctgga tgtcctggca gaagtttgct 12780

acaggggtgg agccctcatg gagaacctct gctagggcag tatggaaggg aaatgtgggg 12840

ttggcgccac cacacagagt tcctactggt gcactgcaca gtggagctgt aagaagaggg 12900

ccattgtcct ccagaccccg gaatggtaga tccaccaaca gcttgcactg tgcacctgga 12960

aaagccacag gcagtcaatg ccagcctgtg aaagcagctg gaggggaggt gctgtaccct 13020

gcaaagccac aggggcaaag ctgcccaagg ctgtgggagc acacctcttg catcattttg 13080

acctggatat gagacatgga gtcaaaggag atcatttttt aactttaagg tataacaact 13140

gcccttttga attttagact tgcatgggac gtgtagcccc tttgttttgg cagatttctc 13200

ccatttggaa tgattgtatt tacccagtgc ctgtaccatc attgtatctg ggaagtaact 13260

aacttgcttt tgattttgca ggtttatagg cagaaaggac ttgccttgtc tcagatgaga 13320

ccttggactt ggacttttgg gttaatgctg gaatgagtta agacattggg ggacttttgg 13380

aaaggcaaga ttgtgttttg aaatgtgagg atattagttt tgggagaggt gaggggcaga 13440

ataatatgat ttggctatgt caccacccaa atctcatctt gaattgtagt ttacataatc 13500

tccatgtgtt gcgggaggga cccagtggga ggtaattgaa tcatgggggt ggttattgtc 13560

atgctgctgt tcttgttata atgagttctt atgagatcta atggttttgt aagggacttt 13620

tccccctttg cttggcactt ctctcttcct gccaccatgt gaattaagga catgtttgct 13680

tccccttcca ccatgattat gtttcctgag gcctccccag ccctgtggaa ctgtgagtca 13740

attaaacctc tttcctttat aaattaccca gtcttgagca gttttttata gcagcatgag 13800

aatggactaa tacaccaggg aaattcacaa tctaatggga ggatcttagc tgaacttctg 13860

aactcttggc tcaagtctaa aatcgtcact tagatgaggg tactgagagt taaatgatat 13920

tctggttgct tggaaaagaa cagacctctc ttctaagctg tctaccagct gcctacttta 13980

cctctctact cagaggtctt ccagacactt catgttcaga atacctctat tcattttctg 14040

ctgatatggt tttgctgtgt ccccacccaa atctcatttt gaattgtagc tccaataatt 14100

ctcatgtgtt gtggaaagga cccaatggga gacacttgaa tcatgagggc agtttccccc 14160

atattgttct cctggtactg aataagtcat ggatctgatg attttataag gggtttcctc 14220

ttttgcttga ctctctcatt ctctttttgc ctgacaccat gtaagacatg ccttttgcct 14280

tctgccatgt ttgtgagacc tccccagcca tgtggaactc ttgagtccat taaacctctt 14340

ttttctttgt aaattatcca gtctcaggta tgtctttatc agcagtgtga aattgactaa 14400

cacaccctca aatatactcc tcatccaacc ttccctttct cagtacatct taccaacata 14460

Page 9 of 33

ctcaactgct caggccagaa aacagggttg tctctgattc ctccctctta attccctacc 14520

tctaatcagt tgccaagtcc tgttgattct cccatacaaa tatatctcat atttattgac 14580

ttttcttcat tttcattacc cctgtcctaa tccagtcacc acctccatac aatattgtac 14640

caactgggcc atctccctcc ctttctgctg actttcaaag actccataag actcagtata 14700

aatgtacctg cttgttcact attttcctac tagagtaaaa gcctgctgct ggcaagcatc 14760

atatttatct tgtctgtcag agggtcacta gaactgagga caacattttg gtacatatca 14820

ggtgattcat aaatagataa ggaatttaga aacctcaata gtgaatgctg acattaaata 14880

gtcttctttg aaatatattt ttttctgttt aaattgggtt tattattttt cctcaattaa 14940

aggatgtaat tctgtgaaaa tttttcttaa tatttgcatg ttgaaaactt tgaatcattt 15000

ggtcaaagaa aatgtaatgt atttcttttt taaatgcata cgtgctgatg tataagatct 15060

gtacaggttt gtccaaggct ccctcacaga ccctatggtc tagaagagaa actatttgga 15120

atatatgaaa ataagatttg agtttatttt ttttaagtag ggtactcatt tattcagatt 15180

aatagacaga agcaaataat tttccaaagt agatgtaaaa aatgtacatt cctataagca 15240

atatattagt tctaattgtt aaattcttcg tctacacttg attttttata gtcattttca 15300

ttttagctat tccaagtagg catagagtgc tcttatgtta tagttttcat ttgcatttcc 15360

ctgtcttatg taatatgcaa ctttttgtat ttttaatcaa tatatatttg tacatattta 15420

tgtggggcac atgatatttt gctacattca tcaaatgtgt aatgattaaa tcaggatatt 15480

tagaatatct gtcacctcga gtatttgtca tttctttttt tttttttttt ttttttgaga 15540

ccgagtctca gtctgtcacc cagtctggag ggcagtggca cattctcagc tcactgcaag 15600

ctcactccgt ctcctgggtt gaagtgattc tcctgcctca gccccccaag tagctaggac 15660

aggcatatgc caccatgccc ggctaatttt tgtattttta gtagagatgg ggtttcacca 15720

tgttggccag gctggtctta aactcctgac ctcaagtggt ccatccgcct cggcctccca 15780

aagtgctggg attacaggca tgagccactg cacccggcca tatttatcat ttctatgtgt 15840

tgggaagtcc cctcttccca ctattttaaa gtatgcaata cattattttt aacaataatc 15900

accttactct gctatcgaag aacttattta ttctaactag tatgtttgtg ctcattaatc 15960

aatatctctt cattcctcct aacccccaac catacaccct tctcagcttc tgatgtctgt 16020

cattctaccc tcacttccat gagatcaact tttctagctc ccacataaga gtgagagcat 16080

gagatatttg tctttattgc tagacttatt tcatttaaca taatgatctc cagttccatt 16140

aatgatgctg caaatgtcat aattttattc tttttatgtc tgaatattat tccattgtgt 16200

atgtatacca cattttcttt atccattcat cagttgatgg acacttaggt tgattccata 16260

tctttgctat tgtgaagact gctgcaataa atatgggggt gcaattatct ttttaatata 16320

ctaatttctt ttcctttgat aaatacccag tagtgggatt gctagatcat atggtagttc 16380

tcttttgaga ttttgagaaa tctgcatact gttttccata gtggctatac caacttatat 16440

acctacaaac agtgttatga gttccctttt ctccacatcc ttgccagcat ctgttgtttt 16500

tctgttttta gtaatagcca ttctaactgg ggtaagatga tatctcattg tggctttgat 16560

ttgcatattc ctggtgatta gtgatattga gcattttttc atatacctgt tggccatttg 16620

tatgttttct tttcagaaat gtctattcat gtccttgccc actttttaat agaatttttt 16680

tgtttttgtt ttttaactgt tgcttgattt tcttgtatat tctggatatt agttccttgt 16740

tgtatgaaca atttgcatat attttcttcc attctataga ttttctcttc actatgttga 16800

ttcatttgct atacagaagg ttttcgtttt tagtttagtc ccatttgtct gtatttgttt 16860

ttgttgcctg ggtttttgag gtcttaatca taataatctt tgcttaggtc aatgtcctat 16920

agaacttccc ttatgttgtc ttctaacagt tttatagttt ttggtcttac ttaagtcttt 16980

aatctatctt gagttgattt ttgtatatag tgagaaatgg agtctagttt cattcttctg 17040

catgtgaata tccaattttc ccagcaccac ttattaaaaa agatgtcctt tcttcaatat 17100

atgttcttgg cacctttgtc ataaatcagt tggctataaa tacatgcatt tatttctgga 17160

gtctatgttt tattggtcta tatgtccatt tatatactaa caccatgctg ttttgtttac 17220

tatacccttg aagcatattt tgaagtcagg tagtgtggtg cctccatctt tgttcttttc 17280

gttcaggatt gctttggcta ctcaagctct tttttggttc catagagatt ttggaattgt 17340

ttttcctgtt tttctgaata atgtattggt attttaaaaa taaagattgc gggggaggag 17400

ccaagatggc cgaacaggaa cagctccggt ctacagctcc cagcgtggtg gatgcagaag 17460

atggtgattt ctgcatttcc atctgaggta ccgggttcat ctctctaggg agtgcgagac 17520

agtgggcgca ggtcagtggg tgcgcgcacc gtgcgcgagc caaagcaggg cgaggcattg 17580

cctcacttgg gaaacgcaag gggtcaggga gttccctttc ccagtcaaag aaaggggtga 17640

cagatggcac ctggaaaatc aggtcactcc cacctgaata ctgcgctttt ccgacgggct 17700

taaaaaacgg cgcaccatga gattatatcc cgcacctggc ttggagggtc ctacgcccac 17760

agagtctcgc tgattgctag cacagcagtc tgagatcaaa ctgcaaggcg gcagtgaggc 17820

tgggggaggg gcgcccgcca ttgcccaggc ttgattaggt aaacaaagca gccaggaagc 17880

tcgaactggg tggagcccac cacagctcaa ggaggcctgc ctgcctctgt aggctccacc 17940

tctgggggca gggcacagac aaacaaaaag acagcagtaa cctctgcaga cttaaatgtc 18000

cctgtctgac agctttgaag agagcagtgg ttctcccagc atgcagctgg agatctgaga 18060

acaggcagac tgcctcctca agtgggtccc tgacccctga cccctgagca gcctaactgg 18120

gaggcacccc ccagtagagg aacactgaca cctcacatgg cagggtactc caacagacct 18180

gcagctgagg gtcctgtctg ttagaaggaa aactaacaaa cagaaaggac atccacacca 18240

Page 11 of 33

aaaacccatc tgtacatcac catcatcaaa gaccaaaagt agataaaacc acaaagatgg 18300

ggaaaaaaca gaacagaaaa actggaaact ctaaaaagca gagcaccaat cctcctccaa 18360

aggaacgcag ttcctcacca gcaacggaac aaagctagat ggagaatgac tttgacgagc 18420

tgagagaaga aggcttcaga cgatcaaatt actccgagct acgggaggac attcaaacca 18480

aaggcaaaga agttgaaaac tttgaaaaaa atttagaaga atgtataact agaataacca 18540

atacagagaa gtgcttaaag gagctgatgg agctgaaaac caaggctcga gaactacgtg 18600

aagaatgcag aagcctcagg agccgatgtg atcaactgga agaaacggta tcagcgatgg 18660

aagatgaaat gaatgaaatg aagcgagaag ggaagtgtag agaaaaaaga ataaaaagaa 18720

acgagcaaag cctccaagaa atatgggact atgtgaaaag accaaatcta cgtctgattg 18780

gtgtacctga aagtgatggg gagaatggaa ccaagttgga aaacatgctg caggatatta 18840

tccaggagaa cttccccaat ctagcaaggc aggccaacgt tcagattcag gaaatacaga 18900

gaacaccaca aagatactcc tcaagaagag caactccaag acacataatt gtcagattca 18960

ccaaagttga aatgaaggaa aaaatgttaa gggcagccag agagaaaggt cgggttaccc 19020

tcaaagggaa gcccatcaga ctaacagcag atctctcggc agaaacccta caagccagaa 19080

gagagtgggg gccaatattc aacattctta aagaaaagaa tttccaaccc agaatttcat 19140

atccagccaa actaagcttc ataagcgaag gagaaataaa atactttaca gacaagcaaa 19200

tgctgagaga ttttgtcacc accaggcctg ccctaaaaga gctcctgaag gaagcactaa 19260

acatggaaag gaacaaccag taccagctgc tgcaaaatca tgccaagatg taaagaccat 19320

cgagactagg aagaaactac ataaactaac gagcaaaata accagctaac atcataatga 19380

caggatcaaa ttcacacata acaatattaa ctttaaatgt aaatggacta aatgctccaa 19440

ttaaaagaca cagactggca aattggataa agagtcaaga ccaatcagtg tgctgtattc 19500

aggaaaccca tctcacgtgc agacacacat aggctcaaaa taaaaggatg gaggcagatc 19560

taccaagcaa atggaaaaca aaaaaaggca gggattgcaa tcctagtctc tgataaaaca 19620

gactttaaac caacaaagat caaaagagac aaagaaggcc attacataat ggtaaaggga 19680

tcaattcaac aagaagagct aactatccta aatatatatg cacccaataa aggagcacca 19740

agattcataa agcaagtcct gagtgaccta caaagagact tagactccca cacattaata 19800

atgggagact ttaacacccc actgtcaaca ttagacagat caacgagaca gaaagtcaac 19860

aaggataccc aggaattgaa ctcagctctg caccaagcgg acctaataga catctacaga 19920

actctccacc ccaaatcaat agaatataca tttttttcag caccacacca cacctattcc 19980

aaaattgacc acataattgg aagtaaagct ctcctcagca aatgtaaaag gacagaaatt 20040

ataacaaact atctctcaga ccacagtgca atccagctag aactcaggat taagaatctc 20100

actcaaaact gctcaactgc atggaaactg aacaacctgc tcctgaatga ctactgggta 20160

cataacgaaa tgaaggcaga aataaagatg ttctttgaaa ccaaggagaa caaagacaca 20220

acataccaga atctctggga cgcattcaaa gcagtgtgta gagggaaatt tatagcacta 20280

aatgcccaca agagaaagca ggaaagatcc aaaattgaca ccctaacatc acaattaaaa 20340

gaactagaaa agcaagagca aacacattca aaagctagca gaaggcaaga aataactaag 20400

atcagagcag aactgaagga aatagagaca caaaaaaccc ttcaaaaaat taatgaatcc 20460

aggagctggt tttttgaaag gatcaacaaa attgatagac cactagcaag actaataaag 20520

aaaaaaagag agaagaatca aatagacgca ataaaatatg ataaagggga tatcaccacc 20580

gatcccacag aaatacgcac taccatcaga gaatactaca aacacctcta cgcaaataaa 20640

ctagaaaatc tagaagaaat ggataaattc ctcgacacat acactctccc aagactaaac 20700

caggaagaag ttgaatctct gaatagacca ataacaggat ctgaaattgt ggcaataatc 20760

aatagtttac caacaaaaaa gagtccagga ccagatggat tcacagctga attctaccag 20820

aggtacaagg aggaactggt accattcctt ctgaaactat tccaatcaat agaaaaacag 20880

ggaatcctcc ctaactcatt ttatgaggct agcatcatcc tgataccgaa gccaggcaga 20940

gacacaacaa aaaaagataa ttttagacca atatccttga tgaacattga tgcaaaaatc 21000

ctcaataaaa tactggcaaa ctgaatccag cagcacatca aaaagcttat ccacaatgat 21060

caagtgggct tcatccctgg gatgcaaggc tggttcaata tacgcaaatc aataaatgta 21120

atccagcata taaacagagc caaagacaaa aaccaaatga ttatctcaat agatgcagaa 21180

aaagcctttg acaaaattca acaacccttc atgctaaaaa ctctcaataa attaggtatt 21240

gatgggacat atttcaaaat aataagagct atctatgaca aacccacagc caatatcata 21300

ctgaatgggc aaaaactgga agcattccct ttgaaaactg gcacaagaca gggatgccct 21360

ctctcaccac tcctattcaa catagtgttg gaagttctgg ccaaggcaat taggcaggag 21420

aaggaaataa agggtattca attaggaaaa gaggaagtca aattgtccct gtttgcagat 21480

gacatgattg tatatctaga aaaccccatt gtctcagccc aaaatctcct taagctgata 21540

agcaacttca gcaaagtctc aggatacaaa atcaatgtac aaaaatcaca agcattctta 21600

tacaccaaca gacaaataga gagccaaatc atgagtgaac tcccattcac aattgcttca 21660

aagagaataa aatacctagg aatccaactt acaagggatg tgaaggacct cttcaaggag 21720

aactacaaac cactgctcaa ggaaataaaa gaggataaaa acaaatggaa gaacattcca 21780

tgctcatggg taggaagaat caatatcgtg aaaatggcca tactgcccaa ggtaatttac 21840

agattcaatg ccatccccat caagctacca atgcctttct tcacagaatt ggaaaaaact 21900

actttaaagt tcatatggaa ccaaaaaaga gcccgcatcg ccaagtcaat cctaagccaa 21960

aagaacaaag ctggaggcat cacactacct gacttcaaac tatactacaa ggctagagt 22020

aaccaaaaca gcatggtact ggtaccaaaa cagagatata caagaatgga acagaacaga 22080

gccctcagaa ataacgctgc atatctacaa ctatctgatc tttgacaaac ctgagaaaaa 22140

caagcaatgg ggaaaggatt ccctatttaa taaatggtgc tgggaaaact ggctagccat 22200

atgtagaaag ctgaaactgg atcccttcct tacaccttat acaaaaatca attcaagatg 22260

gattaaagac ttaaacgtta gacctaaaac cataaaaacc ctagaagaaa acctaggcat 22320

taccattcag gacataggca tgggcaagga cttcatgtct aaaacaccaa aagcaatggc 22380

aacaaaagac aaaattgaca aatgggatct aattaaacta aagagcttct gcacagcaaa 22440

agaaactacc atcagagtga acaggcaacc tacaaaatgg gagaaaattt tcacaaccta 22500

ctcatctgac aaagggctaa tatccagaat ctacaatgaa ctcatacaaa tttacaagaa 22560

aaaaacaaac aaccccatca aaaagtgggc gaaggacatg aacagacact tttcaaaaga 22620

agacatttat gcagccaaaa aacacatgaa aaaatgctca tcatcactgg ccatcagaga 22680

aatgcaaatc aaaaccacaa tgagacacca tctcacacca tttagaatgg caatcattaa 22740

aaagtcagga aaccacaggt actggaaagg atgtggagaa ataggaacac ttttacactg 22800

ttggtgggac tgtaaactag ttcaaccatt gtggaagtca gtgtggcgat tcctcaggga 22860

tctagaactg gaaataccat ttgacccagc catcccatta ctgggtatat acccaaagga 22920

ctataaatca tgctgctata aagacacatg cacacgtatg tttattgcgg cattattcac 22980

aatagcaaag acttggaacc aacccaaatg tccaacaatg atagactgga ttaagaaaat 23040

gtggcacata tacaccatgg aatactatgc agccataaaa aatgatgagt tcatgtcctt 23100

tgtagggaca tggatgaaat tggaaaacat cattctcagt aaactattgc aagaacaaaa 23160

aaccaaagac cgcatattct cactcatagg tgggaattga acaatgagaa cacatggaca 23220

caggaagggg aatatcacac tggggactgt tgtggggtgg ggggaggggg gagggatagc 23280

attgggagat atacctaatg ctagatgacg agttagtggg tgcagtgcac cagcatggca 23340

catgtataca tatgtaacta acctgcacaa tgtgcacatg taccctaaaa cttaaagtat 23400

aataaaaata aaaataaaaa aaataaaaat aaaaataaag attgcactga atccatacat 23460

tgcttgggta gaatggtcat tttaacaata ttaatacttc agatccatga tcatgggatg 23520

tctttgcatt cgtgtgtgtt gtcttcaacg tgtttcatca gatttttgta gttttccatg 23580

tagaggtctt tatccatctt ggttaaattt atttctaatt ttttatagct attgtaaaag 23640

ggattgcctt cttaatttct ttgtcagcta gttcatcatt ggtatataga aatactactg 23700

atttttttgt atattgattt ttgtatcctg caactttact caatttattt atcagatcta 23760

agagtttttt gatggagtct atgtttttct agatatagga ttatgtcatc tacaaagagg 23820

gacaatttga cttcctcttt tccagtttgg atgcctgatt gttctgagta ggacttctag 23880

tacaattttg aatagcagca gtgatagtga gcaccttgtc cttttctagt tcttagagga 23940

aaggctttca actttccaca ttcaatatga tattagctgt ggttttatta tgtgtggtgt 24000

ttattatgtt gaggcatgtt ccttcctagt atttttagtt tttatcatga agtgatgttg 24060

aattttatca aatgcttttt ctgcatatat tgagaggata gatgcaaaaa agcattttta 24120

tgcgaagtca cccagtactg aactttttat tgttgggagt ctttattgct gatttaatct 24180

gattacttat tattggtcta tttaggtttt ctatttcttc ctgattcaat cttggtatat 24240

gtgtccagga atttatccat ttcatctagg ttttctagtt tgctaataca tacttgttca 24300

aaatagtctc tgatactttt gtatttctgt gctatcagtt ataatgtctc ctttttcatt 24360

tctgatttta tttttggagg tcttctcatt tttcttggtt agtctagcta gtggcttatt 24420

cattttgttt atcttttcaa aaaaccaact tttgtttcct tgacacattg tactttttaa 24480

atttttattt tatttagttc tcttatcttt attatttctt ttattctact actttgtggc 24540

ctagttagtt attgcttctc aagttccttg aggtgcatta ttaggttgct tatttgaaac 24600

atttttagtt ttttgttgtt gatatttatt gtgttaaact tccttttgac tactactttg 24660

tgttgtttcc attttcattt gtttcaagaa atttattgat ttattcctta attatctctt 24720

tgacccaaag gtcattcaga gacatttttt ttaattttca tgtatttata cagtttccaa 24780

agtttctttt gttattgatt tctagtttta ttcattgtgg tctgagaaga aacttgatat 24840

aatttagatt ttttaaaaat gttttgagac ttattttgta tcctagcatc tggtctatcc 24900

tggagaatag ttccatgtgc tgacaatgag tactctgcag ctgttcaaca aaatgttctg 24960

taaatatctg tgaagtccat tggcctaaaa tgcagtttaa atccaacgtt tatttattaa 25020

ttttctgtct agatgagctg tctggttctg agagtgggat gttgaattcc tcaactattt 25080

ttgtgttgaa gtgtctctct ccctttagat ctattaatgt ttgctttgca tatatctggg 25140

tgtgatactg cttctcagcc ttttggctaa gatcaggttt agtatctggt tgctctggta 25200

ttggtgcata tatgtttaga actattctat cctcttactg aattgatctc gttgtcatta 25260

tataatgact gtgtcttctt tttactgttt tgacttaatt tctgtttcat ctaatataag 25320

tatagctact cttgcttttt tgcttttgtt tgcatgaagt atatttttct gtctttcact 25380

tctagtctat atctgtcttt tctatctttt acttctagtc tgtatctgtg tacaggtgag 25440

atgagcttct tagcacataa ttaagtcatg ttctttaatc aattcaccca ttctatgtct 25500

tgtgaatgga aatcttagtc tgtttacacc caaggtgatt atcgatatct gagtcttcat 25560

tcctgtcatt ttgttaattg atttataatt gttatgtata tcttttgttc ctttctctct 25620

ctctctctct gtcttattgt ttatcattgc agttgggtgg ttttctgtag tgctaacatt 25680

tgattccttt catttcctta tttgtgtttg ctctaccagt gggttttata ctttcaagtg 25740

ttttcataat gatagatatc atccttttgc tttcacatgt cagcctccct gaggtatttc 25800

ttgtagtgct ggtctagtgg tgatgaattc tctgagcttt gcttctgtga gaaaagactt 25860

tatttttcct tcatttataa aggacaactt ggctgggtat atggtttttt ctttcagtga 25920

cttgaatata ttatcccatt atctcctgga ctgaatgatt tctgctgaga aatctgttag 25980

tctgattgga gtttccttat aagttactag atacttttct tttgctgttt ttagaactct 26040

ctatgtcttt gacttttttt tttttttttt ttgagatgga gtctcgctct gtcccccatg 26100

ctggagtgca gtggcgtgat ctcagctcac tgcaagctcc gcctcctggg ttcacaccat 26160

tctcctgcct cagcctcccg agtagctggg actacaggca cacgccacca tgcctggcta 26220

attttttgta tttttagtag agatggagtt tcaccgtgtt aggcaggatt gtctcgatct 26280

cctgacctcg tgatctgcct gtcttggcct ccgaaagtgc tgggattaca ggcgtgagcc 26340

accgcaccca gccatctttg acttttgaaa gtttggctac aatgcaccac ggggaggacc 26400

ttgaatttta tttgtgtgag tttctctgag cttcctgcat ctagatgtct aaatctcttt 26460

ttgttagact tgggaagttt tcacctataa ttttgttaaa caggttttct gtccctttta 26520

ttttcttttc accttctggg acactgaaaa ttcaaatatg tcatgcaagc tttgttcatt 26580

attattattt tttatgtttt tagtgttcat ctgactgggt tatgtcaaaa gacctgtctt 26640

caaattctga ttctttcttc aacttgatat agtctattgt taaaactttt gaatgtattt 26700

tgtatttctt tcaatgaatt cttcattcca gagtttgtct ttggttcttt gtttttatac 26760

ctgtctcttt agtaaatttc tcattcatat tatgaattgt ttttctgagt tgtagtctat 26820

ctcactgaga taggctttaa tattgttatt ttgaattatc tgggatttca aaaaattctt 26880

attcattgga atctgttgct ggaggattat tttgttcatt tggaggtgtc atattttctt 26940

gctttttcac atctcttgtg tctttgcagt ggtatctgtg tatctggtgt agcagttgct 27000

tgttctaatt ttttttcttt ttctttttat ttgagacaga gtctcgctct gtcacccagg 27060

ctggagtgca gtggcaccat cttggcttac tgcaacctcc gcctcccggg ttcaagcaat 27120

tctcctgcct cagccttcca agtagctggg attacaggtg cccgccacca tgcctggcta 27180

attttttgta tttttagtag agacggggtt ttaccatgtt agccaggatg gtctcaatct 27240

ccagacctcg tgatctgcct gtctcagcct tccaaagtgc tgagattaca gccgatttat 27300

taaattatct tttgtcaggg aaactttttc ttgaaggtgt atctatgctg ccagttgggt 27360

aaagttcctt ggcttttgat tttgggtgca ggcagtagca tagtctctat gatttatttg 27420

actgtaaaca gtatcagtag tatctatgat tccttcagtg gcttagagtg tggttattag 27480

tggaagcctg ttgtaaaatt ttgctgggga cagggatgtg atgtgaatca gtctttagac 27540

cccagtggtg gcagcagtgg gccacatgtg cctctcctaa gctgctcagg acttggtgga 27600

tatgtgggtc ctggtgtgag ctccctctct gaagcaatgc catcacatga tttccaggca 27660

gctctctgta tcagtttcag agaccacatg ggttgacaga cttccccaag gctaggactg 27720

caggagtaca tggtgggaat gtggatggct ggattcattc acttaatctt tcctcatatt 27780

ttagggagcc tctctaggct catagatgat gctagctgag caggctgcct tacttctctc 27840

ttcttcctta ccttaggcat ttcctgtaat ttctctgttg aattcaaaat tatattttag 27900

atgataagtc tgattaccta ctcactccat tggttcttct ctgtggagca ggcaagtacc 27960

agatgcctct agcctaccgt cttgaagaca ccctccaaga caagtttctt aaaggaggta 28020

tgctaaggta gaaccttttt tttgttagaa gttataaaat tccaatggaa tgagtatata 28080

caaaaagaga acattattgg ttcatgtaaa catgatgttg gctattatca gtgcttactt 28140

gttcctgcgt ttgtctctgc ctcacctgcc taacagcttg ttctttcagc cttgctttct 28200

tcatgaagct ggaactgtag ccagctctaa gttcacttca tgccagatgc ataagcaaag 28260

gagaaaggat ctccaattac acctcgcaaa ccgacattca ttctgtgcaa atgagaaata 28320

tctcagggga ggactcaggc ccgacttggg ccacacacac acccattggc cagtcactgt 28380

gaaacaattt tgtagactgg ttgtgagggc agaagtagaa tttaacaacc ttttctggaa 28440

ccacataatt gaaggaaggg ggaaaagttc cccctaaatg attttggacg cacagttacc 28500

agaagaagag ataaagataa ggagaagaaa aaggaaaaaa taaaaacaga aacagtatat 28560

actctaggag gtacatatgg agtatcatgg gaatttagaa gaaagagatt atttcttaga 28620

agaattcagt gaaatctgat ggagtcttta aaaaatgaag gaatatatac tctcaagtgg 28680

ttgcaaagaa catttcagga tagacaatac cagcttgaaa atatgagttc agtgtagatg 28740

ttttaaaaat aagaaaagca gagtagttaa gtgtagttaa ctacactttg aaattaattt 28800

ttagtttatt ttattttttt gagatggaat ctcgctctgt tgcccaggct ggagtgcagt 28860

ggctctatct cagctcactg caagctccgc ctcctgggtt cacaccattc tcctgcctca 28920

acctcctgag tagctgggac tactggcgcc tgccaccacg cctggttaat tttttgtatt 28980

tttagtagag acagggtttc accgtgttag ccaggatggt ctcaatctcc tgagctcgtg 29040

attcgcccgc cttggcctcc caaagtgctg ggattcacag acgtgagcca ctgcacccag 29100

cctgaaatta attttttagt ccacatgaag cagatgagga acatcaaaac catctttaaa 29160

tatatcagtc ttgtagatgt ttgtgaatta aattgattga cagagacaga ctaacaagaa 29220

gttatttcag tgatctatgt ggaaagtctt gagagcctga atgtaatgag aataatacca 29280

agatataagc agaatcttgc actgaacatt gccaaatatt aaatactgtc cctagttatt 29340

tgtcagtgat attttaatga ttattgccat cttcctcttt tttccccatt ctccaaaact 29400

gcattattat aatatacagt attataatat atatattata tatataataa tatatatata 29460

atataatata atataataag gttatggatt aactcctctt aattatctga aaagagataa 29520

cgttcatgtg ctcagcacac aactaagtag tctgatttgg tgttttttat tttatttatt 29580

Page 17 of 33

ttagaaaaga agactttcaa aaggagaaaa atctactgcc aaataactca gcacctctta 29640

cagaaccaca agatgtggaa gaaagtcatt gaagaggagc aacggttggc aggcatagaa 29700

aatcaatccc tggaccagac ccctcagtcg cactcttcag aacagatcca ggctatcaag 29760

gaagaagaag aagagaaagg gaaaccaaga ggcgaggaga taccaaccca aaagccagac 29820

cagtgacaat ggatagaatg ggctgtgttt ccaaacagat tgacttgtca aagactctct 29880

tcaagccagc acaacattta gacacaacac tgtagaaatt tgagatgggc aaatggctat 29940

tgcattttgg gattcttcgc attttgtgtg tatattttta cagtgaggta cattgttaaa 30000

aactttttgc tcaaagaagc tttcacattg caacaccagc ttctaaggat tttttaagga 30060

gggaatatat atgtgtgtgt gtatataagc tcccacatag atacatgtaa aacatattca 30120

cacccatgca cgcacacaca tacacactga aggccacgat tgctggctcc acaatttagt 30180

aacatttata ttaagatata tatatagtgg tcactgtgat ataataaatc ataaaggaaa 30240

ccaaatcaca aaggagatgg tgtggcttag caaggaaaca gtgcaggaaa tgtaggttac 30300

caactaagca gcttttgctc ttagtactga gggatgaaag ttccagagca ttatttgaat 30360

tctgatacat cctgccaaca ctgtgtgtgt gtgtgtgtgt gtgtgtgtgt gtgtgtgtgt 30420

gtgtgaaaga gagacagaag ggaatggttt gagagggtgc ttgtgtgcat gtgtgtgcat 30480

atgtaaagag atttttgtgg tttaagtaac tcagaatagc tgtagcaaat gactgaatac 30540

atgtgaacaa acagaaggaa gttcactctg gagtgtcttt gggaggcagc cattccaaat 30600

gccctcctcc atttagcttc aataaagggc cttttgctga tggagggcac tcaagggctg 30660

ggtgagaggg ccacgtgttt ggtattacat tactgctatg caccacttga aggagctcta 30720

tcaccagcct caaacccgaa agactgaggc attttccagt ctacttgcct aatgaatgta 30780

taggaactgt ctatgagtat ggatgtcact caactaagat caaatcacca tttaagggga 30840

tggcattctt tatacctaaa cacctaagag ctgaagtcag gtcttttaat caggttagaa 30900

ttctaaatga tgccagagaa ggcttgggaa attgtacttc agcgtgatag cctgtgtctt 30960

cttaatttgc tgcaaaatat gtggtagaga aagaaaagga aacagaaaaa tcactctggg 31020

ttatatagca agagatgaag gagaatattt caacacaggg tttttgtgtt gacataggaa 31080

aagcctgatt cttggcaact gttgtagttt gtctttcagg ggtgaaggtc ccactgacaa 31140

cccctgttgt ggtgttccac acgctgtttg ttggggtagc ttccatcggc agtctggccc 31200

attgtcagtc atgcttcttc tggccgggga gattatagag agattgtttg aagattgggt 31260

tattattgaa agtctttttt tttgtttgtt ttgttttggt ttgtttgttt atctacactt 31320

gtttatgctg tgagccaaac ctctatttaa aaagttgata ctcactttca atattttatt 31380

tcatattatt atatatgtca tgatagttat cttgatgtaa atatgaagat ttttttgttt 31440

ctgtagatag taaactcttt ttttaaaaaa ggaaaaggga aacattttta taaagttata 31500

ttttaatcac catttttata cattgtagtt ctctccaagc ccagtaagag aatgatgatt 31560

catttgcatg gaggtcgatg gacaaccaat catctacctt ttctaattta aatgataatc 31620

tgatatagtt ttattgccag ttaaatgagg atgctgcaaa gcatgttttt tcactagtaa 31680

cttttgctaa ctgaatgaat tctgggtcca tatctcccag atgaaaaact gttaaccaat 31740

accatatttt atagttggtg tccatttctt tccaacactg tttgttatga ttcttccttg 31800

agtacttata tacagacctg ctcattatct aaacaatctt accttctaag taaaccttga 31860

ttgtgatttc cagtttttat tttctctgac gtagtagaaa ggaatgttta cattaaaaat 31920

acttttgttt ctcataaatg gatattgtac tccccccttt caaagcatta ttttacaata 31980

attcatggca ttttaaaaaa taaggcaaag ataatacgac aaaaaatata catggtttca 32040

aggcaaattc tccaataagt tggaaaatgt aaaaaggatc aagtggatgc agcctctacc 32100

taaataatta aaatatattt cagtatattt ctgaattaac accaggtctt cattatttag 32160

aacttactaa attgttttca ttttcttagt tttacctgtg tatctccatg tttgcaaaaa 32220

ttactataag tcaaattttg ccagtgaatt taactatttt tctttccttg caattaaggg 32280

gaaaaaagca tttatcttat cttctcatac cccttgcatc taagtactta gcaaagtcaa 32340

tattttccca ttttccaaat gcgtccatct ctaacataaa tattaattga acatagagct 32400

atgtttggag tgagtggact ggcaggacag ttggaagtcc atcacagtct attgacagtt 32460

tcatcaaagc tgtatagtcc aactagtggg gcagcttggc tactatggtg gaagtctcag 32520

caaactgcct ggttttgttt gtttgttttg ttttaaggta caggaaataa gaggaataat 32580

agtggccaaa gcaattagaa catcttcatt ccagaactgt gttcagcaat ccaggcagat 32640

tgatacattt ttctttaaaa ataaattgct attacagcta gacgtcaatt gggataaata 32700

aagggatgaa gatccactaa gtttgtgact ttcatacaca cccagtacat ctcaaaggat 32760

gctaagggac attttctgcc agtagagttc tccccctttt tggtgacagc aatattatta 32820

tgttcacatc taactccaga gcttacttcc tgtggtgcca atgtatttgt tgcaatttac 32880

tacattttta tatgagccta tttataggtg ccattaaact caggtctttc aaatgaaaga 32940

gtttctagcc cacttaggga aaaagataat tgtttagaaa accataaaat caatggtagg 33000

aaaagttgga actggttacc tggatgccat ggttctctgt taaataaagt aagagaccag 33060

gtgtattctg agtgtcatca gtgttatttt cagcatgcta ataaatgtct ttccggttat 33120

atatctatct aaattaacct ttaaaatatt ggtttccttg ataaaagcac cacttttgct 33180

tttgttagct gtaatatttt ttgtcattta gataagacct ggtttggctc tcaataaaag 33240

atgaagacag tagctctgta cagggatata tctatattag tcttcatctg atgaatgaag 33300

aaattttctc atattatgtt caagaaagta tttacttcct aaaaatagaa ttcccgattc 33360

tgtctatttt ggttgaatac cagaacaaat ctttccgttg caatcccagt aaaacgaaag 33420

aaaaggaata tcttacagac tgttcatatt agatgtatgt agactgttaa tttgcaattt 33480

ccccatattt cctgcctatc ttacccagat aactttcttt gaaggtaaaa gctgtgcaaa 33540

aggcatgaga ctcaggccta ctctttgttt aaatgatgga aaaatataaa ttatttctta 33600

agtaataaaa gtataaaaat tatcattata aataaagtct aaagtttgaa attattaatt 33660

tataaaagtg aactaattgt gtgattatca aatcctgatt aatgagaagt gaataatcta 33720

aaaatcattt atattctact gtgggaggta ttcttggtgc ttccatgtca gtatcgggct 33780

ctccattgtc ctcatcattt atagtacttc taaaataata atggaaacct caataagggt 33840

cttgtcctct tctgtcatcc taaccctgat tctagtttaa tgcctttcct gacttatatg 33900

ccatttgtaa ataaacttcc tttcaaactg ttagaacgct ccaataaaac aaagttctat 33960

gaactttcat agtctagaaa aaccaacaag atccccctta tttagagatt attagctaat 34020

gtgtgaaaga aatatcaata gaagttcaaa tgctaatagc tcaattccag ataattgaaa 34080

gctaaaatat ctatgtactt tgaagccaaa ctgagtttat ttcattgatc gaacaagcag 34140

caacacattt tatgaccatt gtcctaggtc gtttgtgtct ttatgaattt gctgatattg 34200

tcagtccatt ttttaaatct tcaacaaaat tcaattttaa gattaagaaa tcagcaattt 34260

taggtaaaga atattccaag attaaattgt tctttttaag aaatatatca gttttaggtc 34320

ctatgttcac acatcaattt gttcatctgg aagatattca aattcttatt gggaatgaat 34380

tacactacaa acaatgagtt gagctaaaaa ctatacataa cttaaattga gatttgcata 34440

cgtatttgtg tgtttcacac caaagaccca tgctcagaga atggtaacat attttccagt 34500

ctatgttatg caccatggat gttaggcaat gtgtgctatt gccaacacat ggtatttgtt 34560

gaaatacatg ttgctttgac cttacctaat ggtgctttta cctaacttga agtgcttttt 34620

gtgttcccac cagcaaatca ttctaacagc aggctaacaa tggtgaagta aaatgaacat 34680

ttttgggccg ggcgcagtgg ctcacgcctg taatcccagc actttgggag gccaaggctg 34740

gtggatcacc tgaggtcggg agttcgacac cagcctgacc aacacggaga aaccccgtct 34800

ttactaaaaa tacaaaatga ttcaggtgtg gtggcgcatg tctgtaatcc cagctaccca 34860

ggaggctgag gcagaatcgc ttgaacctgg gaggcagagg ttatggtgag ctgagattgc 34920

accattgcac tccagcctgg gcaacaggag ttaaattttg cctcaaaaaa aaaaaaaaaa 34980

aaaaaaagaa cattttttgg caaaatatga agacacgaaa ctgaagataa aggcttcatt 35040

tccatagtgg aaatcagtgt ttacctgctg attcaaaaat actgacctag tatcagtgta 35100

ggtaagacca taaaatacag tgtatagaat tatatataaa tggaaaacaa aattagcttt 35160

tgacatatgt ttaagtctct aaaaggaatc cacattttct ttccagatac agctagtttt 35220

tctccacatg tttttaaggt tgatttcctt gacacacatt ggtatccttg ggtttctgct 35280

tccttgaaaa gttgtggagc acagaagaaa agccttctct ttacagataa aaaatgttga 35340

acatgttcat gttctgacaa tgaagcaatg atttcctcat tgctttgcat taagaagtgg 35400

ttctttagtt atgcaaataa ttcaataacc aaaaatgctt caaagagtaa taataaatca 35460

ccataggtaa gtattctcaa gaatgtttaa atttggttta ttttcttgaa gcaaagttat 35520

ttaatttttt aatttttaac ttaaaatgaa acccacaaaa tgacattccg tggtactgag 35580

tattttcaca taatcagaac tgtaaactac tgattatttt catatactca gaggctgaat 35640

attttgatat aataagaact agtatcatat aacagaaaac tagaaagaag gtttaattct 35700

ttttctaata ttctacaagc ctcatgggga aaatgtttta tgaaggattc aaatacatta 35760

gataaaaatt agtgtcaata atttaacttt gtacaaatta tagtctgaga tcttctgagt 35820

tttcctaaga caaaggagtg tattatccag caatttattg ttttgtgttg ttataaaata 35880

aatgttctat taatgtatat tttattttac taagttctaa cacaaaaggc caaatactta 35940

tctttcctac taagaaaaat taaaccttac taatcttgac tttcacagat gaaacactga 36000

ccagtcaata aacaacagcc actgcattaa tctgcccagc atgatcagat agtaggttag 36060

aatttgaaat tcattaataa aaaattaata acaaaacttt gtaagcctta atggtttaat 36120

atttccgtgt cattgaaagt tcctatttca aattaaacaa actacagcag gatatttttc 36180

ctttcctgtt tcaaaacaaa aaccagatga agaaaaaaca aagacaaact cataggagga 36240

aagcatggct tctcttcaat tatacttctc ttactcaata ttagtaacca aaaaataagc 36300

agaaattctc ctctgccata attaaaagaa agaatggcct tcgacttaag ttctagttcc 36360

tcaagcctgc tttccttgta aagtccatgg tactaagaag caaaaaatgc agagctgtgg 36420

attgggagtg aacactacat aaaatttgca gggtatccca ggacacccct cagtctttaa 36480

tacaaaccaa tttagttact ctggagtgaa atgacttgtg gatacactgt tcaaccatga 36540

cttcctttat gacaaggagc acttatacat gtttccaaat gtgaattagc ccttgaatta 36600

gtttgaaaac tgctacttga ataacttttg aagagaaaga ttttctttcc ggtaatttta 36660

ctccctaaaa aaggcaaaat actcagatgt tcaatgtgaa attacaaata gttgtttgaa 36720

gtatatatac ttcaataaat agttctttgt gtatataaag tgttggttca tagcagtttc 36780

cttgtccctt ttctttttaa aattcccaac tgattatggg tcaaaaactc catcttatat 36840

cagcgatgat ttcattttaa ctatttataa ggataattta gtattatagt attgctaact 36900

ttaataattc taccatatct atatattacc tgttagtgag gttttaatgg ctttacttaa 36960

tacatatttg ttcatcagct taaaaaatca ctaaattttt tatactctgt aaaatatttg 37020

catgggaatt ttagcttatt atgtttgctt aactggtgtt tttttcactt atgactgtca 37080

gtttatgtct taatgtattc tcacagaaga atgcatacca tttttgttcc aacaagaaca 37140

atctgttagt tgataagcac tggtaataaa tatcactgaa cccacccacc ccctgatatt 37200
tcttcctcaa gttttctatc atgcaaatca caaaatgaaa attggtgctt ttacaaatat 37260
ttgaaagctc tcaatgcaaa ataataaaaa tgagattctc cctggctttc tataatatat 37320
taggatatgt gactgaagga aatttaatct gaatttagag tgttttaggt tttggttggt 37380
ttgaataatt tgatttgcat tttggtatta aaatgtgctc tgatttctgc gggcatttgt 37440
tctgaccagt cattacctct tcccaacaga aaaaaaaaaa aaaaaaaaaa aactggcatt 37500
ggcaagtttt aattaatatg gcccaaaaat ttgaccttga aggatatttg taaaccagag 37560
agtatttaat taactaaatt tactgtttct gaaaagcaat attttcaaat aactgccaac 37620
caaagtgaca ctatagggat gtcatttgta catgacaatt tttaacaatg taattcttac 37680
cactctcgac agacctgaat atgtttacta ataactttgc caaatttttc aacttctaga 37740
aaaacaagag tgagtctttt gaacttcatc attttgcaat tgaaataaat cttgtccttt 37800
cacctatatc ttccatttaa tatatgtagc aatacttagt atcatcccat cacaagtatt 37860
gttaggcgac ttagtggcgg agaaatgttt gtaaaaagta gaatctgtac agaaaaaaaa 37920
ctagatcaat tgtatttatt tttattattt tttagcttag atactatgtt gatgctccct 37980
ttttgccaga attactggaa gtgcctcttg gttttatata tataatatat aaatattaca 38040
gtaaatatat actcaaaatt attaaaatta tgtcctagat tatctatgtc gatgttatga 38100
gtgaagataa tgtataatat aaaaataatt ctgcagtatt atgatttgtg tagtatttgt 38160
acaatccact tcctccagtc ctctgaaggc atcctttcct cttatatgca aataccatgg 38220
tgtggtacct aacttactct gttgtgagac gacatctatt ggccttccta caatatccaa 38280
agaaccttgt ggggcttata ttctcgtggt cacatctttc tcactatcac taatgaattc 38340
atagccacat ttagtcatca tctaactttc tgcaactaag ttgtgtcctt tctttaatca 38400
aatagcacca aagccaaaat acccacattt ctggcattca gctttagagt cctataaaat 38460
agacagttgt atttctacct aggtttccta agaaattgat tcttcaatct acacctatta 38520
tttccccatt acctatgata tcaaatccca actcctaagc ctgatgtttt caaatccccc 38580
tttgctctgg cctgaaacta ccctctccag cttcctgctc caaaaggaac cttccactta 38640
tcatttatat ttctttatct caggcaaata catcagttga gttagtgcac tttctataaa 38700
catacacttt ctctctctct cacacacaca cacacacaca cacacacaca cttccaacta 38760
tacacagcac aagaagacga gattgactat ctcttcttca ggtgtgaatt ttactcctct 38820
ctcagggttt tcctcttaca atcatgtcat ggctctgagt ggacgggaga tgattgcttg 38880
gcagtctagt ctgaaccttt aacgccatcc ttcaattaag tgttcttaaa acaagtctta 38940
aggaaggaaa atttaaatta catggtgtga gggataataa tgggtaagtt ctaatggaga 39000

ctaacatcca ggccagagaa gagggattat tctgatcatt acctaaaata ggaagcctga 39060
gacatcagat tagtgtgtcc ttgttcacac caattcacac gtagtccagc tttacaggtc 39120
acccccaagc ctgtaaattc taccccatgt ggttcagcag gctttggggg tgctttctat 39180
ctctaaaatt tttggttcga cttgttcaga tgaaataatt tccagtaagg gatactttca 39240
ccagaataca cagtggttcc attaccctgg agccaaggct actatttgga tattttttta 39300
tactttaagt tttagggtac atgtgcacaa cgtgcaggtt tgttacatat gtatacatgt 39360
gccattttgg tttgctgcac ccgttaactc gtcatttaac attaggtata ttttgttgtc 39420
tcatgttact gaaccatcag ggtagaaagt gattgatccc agtttactag gctgaagtgt 39480
ggtttctgat atcaaaacag tggcaagaaa gattgtgttt ggaatgcaaa gaattctgca 39540
tggagccctt aatgcttcca tgttacatag tgagtgtggt gggacactgg agtcacccaa 39600
taaaaacaga accactaaga aaattgacct tgcaagaatt aacttgcagg tcatcctatc 39660
agtaaaaatg ttccaccatg agaagtccta acagaggcaa atgaaaacgt ggaatggata 39720
gtggaaaaca aaagttttat gtcaacattg gcttcgtagt caaatataga aataaatttg 39780
gaatttatcc actaacactt tttaatctat ttacatatgt gtatatctgt ctctgtatca 39840
tttatttaaa actcaatatg aaaattttca aatgtattca aacaagagag aatagtagag 39900
tgaacaccag gtgcctatca cctagctcaa acaattaata tactgccaat ctgttccata 39960
catacctccc aatatttagt acatctcctc tatatgaaaa tatatgaaat tctatatgaa 40020
tatatgaatt tctatatgaa aatatagaaa tatagaaata tatgaatttc tatatgaaaa 40080
tataacatat ccttctatta gtaaatgatt tgtaaaaaaa aaaaagaaaa gaagtgctat 40140
atttacctct aaaacaatta taccaccatt atcatcccta aaaaaaacta acacaaatta 40200
ctcaatacaa caaatatcca cagtgttctc atttctgagg ttataaaact tctaagaaga 40260
ttacaaagaa gtcttcttgt tatagtttgt ttctttttga ataggtgtac gaagtctgta 40320
cattgtaatt ggtcagttca tttcttaagt taaaatatac aatttctccc tcctctcctt 40380
ttttccttgt ggtttttggt tggaaaaaat attgacttat ttgtcctaaa tagattcttt 40440
actttcaatt ttgctgattg tatcttttgt ggtgtcattt aacttgctct tctcttccct 40500
atatttctta tggactggta cttaaatcta atctgagatt tgataagatt agttttcttt 40560
ggaagtaaaa aggaggacta tttcaaaggt ggtgtttcaa gcttttaact tccatcagga 40620
tgttgaaaat ggcttgagat ttttggtgat attattggtc attgattacc tttcgctaaa 40680
ttcattaatt cattagaggt ttgaaaatta taatactctc acttataagt gaggctaaac 40740
attaggtaca catggacata aaaatgggaa caatagacat ggcacattac ttgaggggga 40800
gatggaggag aaaggcaagg gctgaaaaac tacctattgg gtaccatgct cactatctag 40860
ttgatggaat cattttaccc caaaactcag tgtctcacaa tatacccatg taaaaaatct 40920

gcacatatac tctctgaatc taaaattaaa aattataaaa ttaaatatct aatagcttaa 40980

ttctactaga aattaatcta cttatttata atatagaatt ataaaaactt tatagcgagg 41040

gtctgaactg atcatattaa cagaaatttt tcaaaatggt taggaatcat ccttatacaa 41100

taactacttt ctcaatttct ctactgtgcc acaaaaacca agctatgcaa ataaaatcca 41160

ttagagctct accaatctaa aagcaattga aaattagtat attttctaca tttttttctt 41220

taacctttgc atttgactca aatgagttta aggattttgt atttttaata ttgtttttat 41280

taaatctgtt ctagaaataa atttatcatc tctggagaat agtctctatt ttcagactat 41340

ttgttggact aaaccaaggt tgcactggta ttatttcagt aaaacttcaa aaccttacca 41400

aaatcctttc aggatacttt aagattgaat tacatttcaa taaatattaa ttaccctaag 41460

aaaagaaaga aaattataat attcttattc tctcatcctt ttttgtttat tagttggaat 41520

tctatgaaga gaaatttgtc tttactctgt aataactcta aggaaagaat ggaatagaaa 41580

agctaggata aacacacaat ttctccctaa ataccactag ttttaaatct attaatattt 41640

aagttgtgtt tagaattact ccaaagtgac catgtgatgt tttaatagtg tttttaactc 41700

ataaattttg tgattttata tgtttcaacc attactattt tatatgtttc aaccattaca 41760

attattctta ccaatgctca aactttccta tatttgatta gcagaaacct gttcaggctg 41820

gcttctgaat cctattggca caatgtagtc ttcaaggcat cccagctctt ttttatgaca 41880

agatattcca ggcctatctt atatctttac acctggtatt agtcacatat caaggatcca 41940

tggtgctttt atttatggtg ctagttgcac ttattactac ttagttgatc attgtttcta 42000

gaccttttca gtggagagag ctaatacaca cacacgtaga tatctatatc tatctatatc 42060

tatatctgta tctacatatc tcctgtatat atgtatacat ttttccagac tgaaaaactc 42120

aactcaaatt tgggtataca aaatattccc taacctcatt ggatcttata ttatagatta 42180

tatttaaagc catgacacca gataaggtca ttaaggaagt aaatatagac agcaaagaag 42240

atattccaca gactgaattt tggaatattc tagcaatgaa gactaagagc aaatgaccac 42300

tgaagtagga ggaaaaccag gggtgtgtgg tgaaaccaag tgaataaaat gtatcaaaga 42360

gaagggcaca ttaatgacat acaattctgc taatacatca attaaaatga ggtctggata 42420

ttgatcactg gatttattag tgtgaatgcc tttaatgttc ttgatgaaaa tatttgcatt 42480

ggagtggtaa aggcaaaacc tgattagagt tggtgaaaaa tgaactggag gagaggaaat 42540

atatacaagg aatgtgacta aagtttgggc ttagaaacat ggactgtagg agcaagaagg 42600

aagacagcta atagcaatgt acatttgggt agagagtaga tgtcatgagg acagagtata 42660

atatttctcc tgatatttct gtttagttag tgaatttcct agacaagatc atcagctaat 42720

aatcagggtg cagccagaaa tgaagatttg agagaggtaa agtgattggg tcattaaaaa 42780

gtattacgtc tagcaatgcc tctatgactc agtgagttta gtggttataa atataaaata 42840

agccctaagg gcatgttact gagttgttct tcaatcactt aaaacagtgc ggatgcaaat 42900

actaagaaga aagaaagtgg atttatctga ggtgagcttt gcctgggaag atcaataaat 42960

tgaaagagaa acaggagaat tgagggtata cagaaggaag tgattaaaat aattgcatga 43020

aaaaattcaa gtaaggagga aaatgaaaat atgagtgtac tggcttgaat tgtgtctccc 43080

aaaaagatat gaggaagtcc taacccccgg tacctgtgaa tgtcaccttt cctgaaaaga 43140

gagtcttagc agaccttatc aagttaagat gaggtcatga gggtggtccc cagccaaaga 43200

tgactagcgt ttttttaagt acagaggaca gagacataaa gggagaagat ggccatgtgg 43260

taacagaagc agctgttgga gtgatacacc tataaaacaa ggagttcagg ctgtcagcaa 43320

acaccaggag gtggaagagg taaggaggat tcttccctac aggtctcaga gggagcatgg 43380

ctctgctgac accttgattt ttgacttctg gcctccaaaa gtgtgagaca ataaagttct 43440

gttttaggtc actcagcttg tagtactttg ttagtgcagt cttaggaaat gcattcattt 43500

atatgttgtc tgtggttaat ttaccaccag aatggcagag gtgagtggtt gtgatagaga 43560

ttgcatggtc ctacaaaacc taaaatattt cttatttgtc cctttataga agtttgttaa 43620

ctccctcatc tagagtttgg aaatgggaat gaaaggctga agtaggctac aatccaatgc 43680

aaggttgcac ataagaagct ctgaataagt gtaaacatta tgtgcattca aaacagtaac 43740

attaactgtg gactctggaa ataagaaaag gaatcgtgtt ggagagtgag actgagccag 43800

ttgggaatag acaaaattac attctcaagg gagaatttca tttcttggtg aggaaaatta 43860

tgccctttgg gctggtaaat actgtctcct cttgaataaa acagtggtct ggccacctgt 43920

tagacactga gaagtctaga ctaacactca atgtattcgg atgtttgctc actatttccg 43980

atggaaaagt ctggtttgat tgttaaagta aaatatttgg cctttcagag tgtgaaaaca 44040

ccattgcttc attgcaaggt ttaatataaa cccatttttc ttgcattttc ttttctttct 44100

tttgtagtaa aatgtgcttg aataatatta actattctga ccattatgtg aactaaatat 44160

atatatatat aaaatgatga taccttccgc tcatttgcaa tgtgcttttg aatacattat 44220

ataatttaat ccttaaaaca gccctataaa tctgtttttt ttttttttaa acaaaaaaat 44280

tctgctttac aaaggaagaa gttgagactc agtaaaatta agtgaccatc ccagagtctc 44340

agccacttaa gagtagggtg aatcttggat aattctggta gcaaatttat ttattggtag 44400

aaaataaatg caaactacta ctgcttaaga tcctctaaag tttttctctt ggaaaaagaa 44460

tgtattagaa ttgtggactt tctcagtgca aggatttatt tatccttgga attaggcaga 44520

ataaacaatt gaatgccaaa aattcaacgt gtttcttttc tgttttcaaa actcatagaa 44580

attgtaaata tgagcaactg atcatgtctc tgagaattaa ctctttttgt acagagcgct 44640

gcagagcttt gattgtgacc tgatacttgg tccttaaaga ttctagaaca ccaatggaaa 44700

Page 16 of 33

agaaaaccat gtagcaaatt agcccaagaa ccgttaaaga caaaacaaaa caaaccaaaa 44760

ccaaaacaca caaacaaaga aaacacaagg atcccagtga tgtccctaag gaatagttgg 44820

cttaaaaact ttctggaagg aggaggggtc tctgttataa acttccgccc tcctctggct 44880

tctgttcctt accctctgcc ccctgcggag ttgtgttttc tgggaatcag caagtaagat 44940

cacaagtggt cttttctttt attcactctc tcccataaag aaagctttat cacgtgtggc 45000

cttaaacttg cagcaaattg caaagaaatg gctcaaaagc ttcagctctt tctgtgccct 45060

gggagctgag atgcacgtca gtggccttgc cagcgtggcc aattctctgc tgactgccag 45120

aaaaaagagg ccaggaagaa agaggaaaga gaagagatcg ctcaggggtg agaccatgcc 45180

cttcatcttt tcttttccct aatctcctct gcttgtgtcc acccacactc tccccacctg 45240

gcaaaattgt tcaaaattgc tgtggagttt acctcagttt cctctttcag tctgtggtgt 45300

gtggtccatc ctcttgctga gcacattgaa aggaactggc tatctttgat ctcttcctcc 45360

aggtaagact tggttctctc attttatatt ttgtgactaa tacatcagat tcttatggga 45420

gattaaccct taatttctgt gcgatttatg ttttatccat aaaaatatca gtgaaaggtg 45480

atgatcccct cattagtttg atggaaataa gagagaagat tggttaaaga aatcagataa 45540

actcaaattt gagttcaaat tttgagtctc atgcttataa attgtttagc ttttgacaag 45600

tcacttaatt gctttgggcc atctaattta aaaaatggaa ttctaatatt acgtttgttg 45660

ggttgtttta aggattagaa aaagtacgta cattactcaa cacaaaaata gtacttatta 45720

aatctgaatc tgacacagat aatttgtatt aggtgagaaa ttagtttcta aacaacacac 45780

actcctctgc agcaagtgcc gtgaggctat tttttagttt gtctgaaggg aggcatcctg 45840

gtgtccagaa gtcaagccag gacagaggtt ttcctcacag gagatggcca agagaagaga 45900

gggaggagcg tggttgcctt taggactttc tctgtggcag tccttgggga ctgattgctg 45960

gacagagtcc gagtcagttg cagagcagag gggttgagta catctgcggg atctaactac 46020

aaccttccta agccacttgc tctctccatt taaaatctgg agggctgatt ttgaaactaa 46080

tgaggactga ttattcaatg tttttgaacc aaaggaagag aaactgcttg gcttgttttt 46140

ctttggcctc cagaataagt tatgctccaa acccagggta ttggatgaaa actgtcgtat 46200

gtcttttcct ggaagaatct attcaagtgg ccagagatca attaaggatt tgccaaatgc 46260

aaataattct gaataactgt gtactcttgc tctacttctg cagttgcaaa tgaagagaga 46320

gagcctgcag gccccaagga tctttccgtt catgtcattg agcttccttc cctctctttt 46380

tacaggggat gaactgtagc ctccttggaa catactacac ttgcaataat ttgcgcatta 46440

accatcaaga cacttcagta aagctatgca gtgccaatag tttaagctgg atcctatttt 46500

gccctaacac aaagtgaact gagatgacta tatttatatt tcattatact tttgaatttt 46560

agttacagtg atttttgtgt caacacctcc atatattaga taacttaaaa tgtgctatag 46620

tatctcaagt ttttcagtag tgtttttaac ataattattt tttctctttg acattagcat 46680

tctagatgta acactttgga tcttttaaag ataaatttag gataaatgat agcagatttt 46740

attattaaac aatttatttg tattttctgg tcactgactt taatattatg actgatgaga 46800

aataatatga tatctgaatc cccaaaaaca gaaggccaat ttgaattata taatttaatc 46860

ctttgaagaa tattgcaatt taagccagaa atttaataat tatccgtttc ttattttaaa 46920

acctttctgg tttgtatatc tcttgcttgg caatattttt actcatagtt ttgtagtaaa 46980

gatacctatt taacattgta taactctgca tttcccaaac cctgttttcc ttgtcctggc 47040

aaaatactgt tctagaaaag tagtcctagt gactcagtag atataggaag ttggctgttg 47100

gtatcagata ctgttaagga agaatctcta tattcttcat ttcttaaact ttgtttttta 47160

aatcagtaat agatgtcgaa ttttatcaac tgatgttttt catgtaatga aattctattt 47220

tttattttga attattcatg tggtaattta ggtgaataaa tttcctgtaa ttaaccaatt 47280

gttgcatctt ttagaataat tctaatcata gagcaagagt taatttggat ttgctagcat 47340

ttacttagaa tttttataat ctatgggctt gatctgaagt tttcctttct gtgctatctt 47400

tgtgctgttt tgttaccaag ataatgttat ttttgtcaaa ctaatggaga caatgattat 47460

ctgtttccat tatcggctat ctttcctcaa agaatatccc ttgataaagc acttctggtg 47520

catcatctat ccttagtatc tttgtttagg gaatagtttc agagtatttt tgataatgtc 47580

tttcttggct attttctctt ctggttttta tcaatatagg taatgaatat ttgctagaaa 47640

cttatccatt aagtcaagat attaaactta cttcaacatt cgatgcatct tttatgtttt 47700

tatactccta aatatctgtg ttttatctca ttaatattat gtatttatat ctttgttttt 47760

tgtcttgatt aacattatta gctctttgtt ttgcttattt tttgttttta aatttttctc 47820

tttcactcac tcttcttttt caattaaaaa attaaagtta tgaatgctta taaaatgaat 47880

ccaaaagcct aataataatt tttttgaaac cagacccttg tctcatcctt tcctttagtc 47940

aattcccact ccctagagga atccacttaa tttttaaatt gttgaagtcc tctgatgttt 48000

taacttcata ttattaatac aaagcaaatc gtttatacta ttacttgatt tttcagtttg 48060

agaaagtatg aattgaaatg gtgagggatg aggtttagtg aacacaaaac aagcattgca 48120

taaagtcaag gtgatatact cagtcagaat agatttctgc cacaatacat gaaatttgac 48180

tcctaacatt attcctttag gaataacatt attccactcc tattatgtgg atatagaagt 48240

cctctattaa tgtacaattg ggtcatctga taatattagg gagattgaga aatactttgt 48300

ttgttgaatt ccttgattta ggttagatta tcaggcaggc actgagccat gtggaatcct 48360

aaagaagtag gggcacagga tatgtaaata ttgatgtgaa cagggagaaa ataggttcag 48420

tgtgtgatcc tgtcctgtga ggggcggtgg gaaattgagc atgggaagca aggctgtctt 48480

Page 17 of 33

ttttacttta aggctgatca aagactgttc tttcacctga attaaatcta aggcacaaat 48540

catgttgctc ccttatttat caaacaaaaa caatacacaa attggacctc ttcttttttg 48600

tcatatttta aggctcacat tgggtgctac aggaatcata aggtggtaca gggacctata 48660

atgaatgtaa aacttctatt tgttttgcta agaagataga tttaaaggct ttaaagactt 48720

tatcctaatg aatcaatatt caaatagttg aattcctctt cttcctttcc tgcttgctcc 48780

tagtttaaac tggcaagacc acaaagttag caaaattctg cctcattatt tattccaaag 48840

gcaacactta aatttattct ttgccataat gaggcttaaa ctgggcagat tgagaaagaa 48900

tgctggctac agagttccaa tagcaggatt agttaatcag atatcctggt tgattttaat 48960

tgaaaacatt tgccaactta ctgtgacaac aaaaggtaga ttcactttaa attgcgtcta 49020

tagaattgtg gtatactgtt gaataaaaaa atttaataaa ttgcgtagta tttatttatt 49080

tttactttaa aaactaactt tgacagatca gagtcaagga atgtgtttat aatggacact 49140

tcatccaaag aaaatatcca gttgttctgc aaaacttcag tgcaacctgt tggaaggcct 49200

tcttttaaaa cagaatatcc ctcctcagaa gaaaagcaac catgctgtgg tgaactaaag 49260

gtagagcaac caagaagtaa atagtgttga tgctcttagt tttctgtcca gatatcatct 49320

atataatgaa gttagaatga gaattgtttt ctcctttaaa aaaaaaaaaa gatcttagat 49380

gcaatttact aaaaccacac cttgccaggc ggtgcaagca atctctacct gaccatgtgg 49440

gttaaattac tttcctgggc tcatttaaat gcccccccaa acagaaaaga gagaaatagt 49500

agaaactagt atttattgaa cttctgccat gtgccagata ttaagccaag cacagatata 49560

acatttaatt tgtgaaacat cacattactc ccattttgca aataaaaatg ggaataatgt 49620

tacagagtag ggagaagagt tgcttttttt tttttttagg attgttgtat caaacaatag 49680

tttttagttt tttgaggaac ctccatacta ttatattttc caaagagact acatcaactt 49740

atgtaaccac taaccatgta caaagattcc cttttcttta cattctaacc agtttttatt 49800

ttttgttata gccaacagat gtgaagtgat atgtcataaa gatttttatt tgcatctcca 49860

taatgactag tgatgttgag cacctttttc atatacctgt tggccatgcc ttcttttaag 49920

taatgtctat ttagattatt tgcccattgt taaaatcatg ctatttttct ttgctattta 49980

gtgatagaag tttctttttt ttaattattt tttaattatt atactttaag ttctagggta 50040

catgtgcaca cgtgcaggtt tgttacatac gtatacatgt gccatgttgg tgtgctgcac 50100

ccgttaactc gtcatttaca ttaggtatat ctcctaatgc tgtccctccc cgctccccca 50160

atcccccgag aatagttgtt tcttaaggga tgctttgtgt gctcctattc tttctttata 50220

caaatattca ctcaacaaat ctctgctttt ttcatataaa tgctaacctt ccctatgaat 50280

gttctatcac agacccatgt ctatcaaaat atataagcta gatatcaaag acatgttttc 50340

cattgagaaa aatatttgac tatgaattta ataagtataa acataaacac aaggaataat 50400
agttgcactt tttcagtctg tacattatgt tcccggctgg tgcatgcacc accctgtgaa 50460
atacataatc ccaagtgctg gacttgttat aggatgtcaa gtgtttggct tatggtttca 50520
tttactttct taccaatgaa tgtctcacca tacactgact cttcaatgta agactccttt 50580
gagtgcccct ctggagcaga gtggaagaag acaaaagata attcaataca catttcgcat 50640
aaaaaccaaa tatatatttt aaattccaag agaatttgaa ctgttgaaaa cttacagtgc 50700
ttaaggctga tggaaacctc gaacagtgtt tgaattaaag tttcagtgat aataaagttg 50760
atattataca ttgttctttg ttgtttgttt tgtatttgtt tgtctattta cttagtttcc 50820
aattgtgtgt caagctggag tcagactaag tgtgacctgt catattttcc aggtgttctt 50880
gtgtgccttg tcttttgttt actttgccaa agcattggca gaaggctatc tgaagagcac 50940
catcactcag atagagagaa ggtttgatat cccttcttca ctggtgggag ttattgatgg 51000
tagttttgaa attggtaggt attacagatg cctgacttta attttaagcc caagatcttc 51060
taggtgtggc taaagaacac cttctgcatt ctattgtctg ctgggatcag actctattca 51120
taaaatcttt tttttttttt ttggacagaa tttaccatga tcttattcta caggagaggc 51180
aattttctca gagcaatcaa cagtaaattt ttctccccag gtgtttggta ttgtatggtt 51240
cacacaattc agagttctac atcagagtaa gtgactttct tggaggaaag ggtgagatgc 51300
taattggtat tttgagttat aaatatgtag aagtatcttt acagaagaac aatacaccct 51360
gggagcacta tgattcaatt ttagaggtgt ggaataatcc aaatgcgctc tacaagaatt 51420
tactgcaatg atgaatagaa gttacatcat aaaagggatt tttccaccca gtggtctaga 51480
aagaagaata aagggattct attaccaaga acacaagatc agagcaggca ataaaaattc 51540
aaatctcagg tgcctttggt tagcaattga ctacagactc cttagaaatt aaaaagatcg 51600
agtaatttga gtcactgttt tagaagtaat ttaaagagga taatcctagt tagtctgctt 51660
cctgaattga taaactaaaa ggctttaatt ttttgctgaa gtaaacgaaa tagtaccctc 51720
tctggtcctt aaggcttgtt tagattcaga tatacaccat agtaaaacct ctaactgctc 51780
caattttagc tttacctggt gaactttcag aaaagtgaat aaatattttt caactcttta 51840
acaatttatt tcttttcata catactatgt gttttggttc ttactctgaa tatcacagaa 51900
aaaataaaat gaataagtaa ccttttgcct tcaggcattt gcttcccaaa ttcatctttt 51960
gtcatccttc cagaatgaca tttcttagtg ccaaatttta atgtccacac aaataactgg 52020
gcaatctttt aaaaatcttt ttttaaaacc tgaattggta ggaacaggat aaaattctgc 52080
atttctaata agctcctaaa tgatactgat gcttctagtc agtagatcac attttgtgta 52140
gcaggtttcc aaaacatctg atcacatcaa tcttttatgt aatttgctta gattggcccg 52200
gatggttgat ttattccttg agccctgttc tactgatggc cctgcactga gtgaagggcc 52260

Page 19 of 33

aaagagaatc aaggtaaagg gtgatacatt atatcatgag agaaaaacaa ggagaaagag 52320
gtaactggtg cagataaaag aatagagcag atatgagcag gcaaaacatt atatatcatg 52380
atgttttgat gtttttctca ctctagatta ttatataaaa atattttaga gctgaagagg 52440
catagaaatg atttggtcca aagttttgtt tgggctccca ggtcctgatg caacttgaga 52500
gacaaagttt atatttgata gagagagtat actaagtttt cacaggaagt agcagaaaac 52560
catgactcca aattcacatt aattcttcca gaccattcta ggtatataaa ttatttattg 52620
gattggatgc cttaaaatgg cttaaaagtt ctcttacttc cattgaaaac ctttatccca 52680
aacatcacag gtttgcccta attatttgag ttacataaat ttccagacaa atgacttcgc 52740
tgaacataat cttaatttac ttcttttgcc tctttttgca ttccttcttt tcaagattct 52800
agaacaaata tcatcctccg tctttatgtt atttgtatgt ggcaactttg ttgaaattct 52860
attagtttta attgattttc catggaaact gtagagtttt gtggacaaat tgtctaaaac 52920
aaaatataat ttcgtatctt tctttgcaac tcttagattt ttgttgttgt tgttgatatt 52980
accttattac attagcctca gaactgtgtt gaagagaagc atttacaatg gacatctttc 53040
tcttgtgttt gaccttgatg caaatacttt taaaatttta gtattaagca atatgcttgc 53100
tttgtggttt tgatagatac actttaaaag ctggtttgct cagatttgtt ttttcctaac 53160
ataaatcaat ggttaatttt atttagtact tttacagtgt ctattaagtt aaccatggaa 53220
ttattcccct tccatttgtt atatagggat ttaattaata atttattgtt agttggtggt 53280
actcttgtac tactataatg aactgcgctt ggttgggata tttttatttg tatatactat 53340
tccgtttgct aataatttgt ttaggatttt tacatctatc ttcataatca agtttgggct 53400
ctaattgtct tttcttatat tgtccttgtc cagttttggt tttaagaaaa tagtagtctt 53460
acaagatgaa ttgagtagct ttcttttttt tctgttatct cgagtgtgtc tgtgtgtgtg 53520
tgtgtgtgtg tgtgtgtgtg tgcatagaca gacagaaaaa gagaggagag caagaatatg 53580
ttactttaaa gtttggtaag acatatttct aaaactttct gtacctgttt ttttgtgaga 53640
ggctaatgat ggtagatatg gaataatttt tatatccaat ttcatttttt aaagattttg 53700
tatcatcatt tatatttcaa atgtatctcc tgacaactca gaaaggatac taaggaagca 53760
aaaactttga tagatacagt atatatatgg tatatagata tatatatata tctgtattac 53820
aggtactgaa ctcatattgt cttgtatatg acatgcccat taacgtaaca cagaaatagc 53880
ttgtaaacag aagtgtttat tgtgtggtat tggtggagtg taggcgagaa taggagagaa 53940
ttacctataa agtgaatgtc aactttcatt ttatgttggt tcgtttattg tggtacatta 54000
acaaacatat agatagaatt atcaaaataa tatgatacac aatgtcttct actaaatact 54060
taaatttcaa atgagacatc ttgtgctaaa ttattcgttt tttaatatga acattaagaa 54120

gagcttaatt cttcttggag ccataaagac taggaccact gtgcaagtca tgtttctcac 54180
tatcattctt attatcctca agcccacaaa aattactgct ttatttccag tttactaaag 54240
ctggcaatgt gcgtgaacag tagtaaatta gatgattgct tacagatgta gggaaacatg 54300
ctttgctcaa aaattggaaa tgaggagaac ttcaaagcaa ggccacccat tagccatcac 54360
aatcaattgc tgggaaattg ttaagatctt actgtattta gcatggtaca tcttaaaaac 54420
taaatatctc aaataaatat agctaaagct ttaaagcccc ttgaagtcta aaaccctaaa 54480
caatgtttat caaagcatgt tctaggaatc atctccatca acacacttga gttagtatta 54540
caaatgcaga tttcttggcc tgacctcaga tccatggaat cagaaacgtt tggtacttgc 54600
caagaatcgc atttttaaca agttttcatt tccttaaccg tttcctgtca gtatttttac 54660
gcacagcaaa gcgtgagaaa tattacttga aacatgttta atcacttatc accatcatca 54720
actaaaagta acgtgtcaca tgcttcatta gagacatttt agaaaaccat aatttctact 54780
ttcaaaaaaa cccaatattt ttaaaccatt tttcacaaaa gagataaatg ggcacatttt 54840
atagagattt tgtatttcat aaaatttgct ttataggcat atgttgaggt tatataattg 54900
atgtacacct gaagtcgcct atgtggaata atttttattt ctattttgag tcttaatctt 54960
tttgtacaaa gtttattaac aaatcataaa gtatcatttg tgtacatgtg tttcatcatt 55020
ttatttcatt taaagtacag ttgtgtttaa tttttctata aaagatatca gctaaataat 55080
ttgtaatgtg tagtccatga gcaattagaa cactaataga agttttcaca taaattttaa 55140
tgaataaaga aaaaataaga agtttacttc aaatttacta aaaaatattt gagagggaag 55200
ctaaagggag ccttctagaa tttgacttac tgataaaata ttttacaagg acaaataatt 55260
aggttgggtg aagtgaggta gaaggattga attaaaatat ttcgataaag ggataaggtg 55320
attgaagtaa tgaacatgag aatttcaagg gtcatctcag attaaaggaa aaaaattaaa 55380
tgatgcagag atggcttagt taaaagaaaa cattgcttgg tctgctgtat acattcattc 55440
agttatgctg ttgacttctt tctaagttct ctaatttatg atgttttatt cttttcctca 55500
aagggaatct cttagttata acatttgtta gctactttgg agccaaactt cacaggccaa 55560
aaataattgg agcagggtgt gtaatcatgg gagttggaac actgctcatt gcaatgcctc 55620
agttcttcat ggagcagtaa gtctaaagca atcatctttt ccttgccttt ccaaacaact 55680
gcatttctcc cttttatgat gattattaat tatgcaaatt taagcttaac ccatgataac 55740
tttgtcttac ttttattaca ttctctttgg ataaaatttc acaacaattt tataattttt 55800
cttctgacaa taaactgtaa aaaattgata gattactgta tattgatatc aacaagatta 55860
aagaaagatt ttacctacat gttttgagtc aaatgcttta atttagctgt aggggcaaaa 55920
tatataattg aaaataaatt aacatggtaa atgtcaaata ggtaattaat aaacactgca 55980
gacaataaat tctggattat ggaacactgg ctgctagaca attaactcag ggatatatta 56040

tcaactcaaa caagtattca atgtccatac agtatctctt agacctttat caatggaagc 56100

agggtgtctg tatcctctaa ccctaaagtc tacttttggt acttttgtta atacatgcac 56160

atgactttag tagttccaaa gatttttacc tatgtcattt ttacccttat agcaacctgt 56220

ggggtagaag aacatagttt atctttttta ctttagagct aaggacagga ccccatgact 56280

tgaaaagttt gatgactttt gtaaatttag gtgtcttgta attatggaat cagaacctaa 56340

aacaggtcag ctgacttctc aggcagcagt tttcatacat atccatgtca ctgcccttct 56400

ctgtgtttat ctattgctga taaaagaaat gactaccaaa aggttttgga gtaagagaat 56460

attttagaag attatatttc aagtgatact ttattgtcca ctctagatca ttgtatcagg 56520

acagtttaga gctggagaga cttagaaatg atctagtctg aactttttct tgaatttcta 56580

gaccacttat gtaagtacag tcagattttt gacctccctg gccaatctat caaggtaggg 56640

ctcagaattc tctgagtggg agcttttctg cccatttgga tgaattccca ggaccctaaa 56700

tttctacttg gagggagttc tgtggtttgg gcacttctgc ctgctctttg gacaacatcc 56760

tttcataggt cagaagtttc cttgatcatt gagattctac aaaaatgtta tgtatgttca 56820

gcctgtagaa agaatgggaa ggagaagaat gagaaaatgg gtctccaacc tgcctactcc 56880

ctgcttgatc tttagttgtg ttgctgttat ctgttgattc attgagcttc catgtgacag 56940

tttgctcagg gggaaaagca gttccactac tgaaaaaatt aaaaatgtga aagtagtcac 57000

ataggataga taaatccatt acaccagact taaatcactc tgcagtggat cagtagctct 57060

tctatgtgac aatggctaca ctacttaatt tttctaggtc tcagtttctg catcttaaaa 57120

caggtattaa aaataattcc tagctgataa tgccattgca aatattaagt gagataacac 57180

aggtaaaatg cttatcaagg ttaccaacct aactagtagc tatgtcatca ttgttgtgtg 57240

ttatcaatcc ttttagctaa ttaagccacc tggttttaac cagcaatggt tttgtcttaa 57300

agctgctgta tacagatgaa taattttatt actttaagtt agtgacaaat atcgaatacc 57360

cgtataagaa aatcagagaa aaaaaaatgt attaaaaaga aggtcggaca accagacagg 57420

ctagtgttga caaaggatat caaagggaca gagacagatt cttatccaac acttttgaca 57480

tggtagagga tatatttgtt tatttattat ttaatatatt tgccaggtac aatgccaggt 57540

aaaggagcta tttctgcgag aaaacaaaat agaacaaaaa cacacacaat tcttccttca 57600

tgaagcttgc tctctactga ggattatcac tctagtttct ttccaccttt acacaacatt 57660

agtaactaat tgggtactta tccaaaatat gattttcgtt ctctaagaga taactatgct 57720

ctgtctcctt tgcagaaata tccttaatgt ccgacaaaag ctattaatca tggatagcaa 57780

ctattgaggg cctattccta ataggctgtt tagttttatg tattagctca tttaactcat 57840

taatttagca agtgtatata tatttgtgat atatgtatat atatataatt tttctaggca 57900

gtaaagaaag agtagtgaca aaataatctc tgccctcaca gatcttctga ggaggagaca 57960

gaaaataaac agataaatac agtatgtcag agaatggtaa gaactataga gaaaaataac 58020

ccagtaaaga cacataagga gtatgtgtgg tgggttgggg gtggttgcag tatcaagtag 58080

ggtagtcaac gtatggctca catagttggt gacatttgaa gaagacctga taatagtaaa 58140

gaggacagtc atgtggatat ctgggaaaga agacttctag cctaagtgaa cagtgaaggc 58200

aaagtttctt cagcaggagg aagtctggtg catggatgac ctgcaaggag gcctgtgtgg 58260

ctaaaatgca atgaccacgg agaaagcaca gaagatgaat gctgagaagt aaaagggaat 58320

gagagaatat gtcaacgttg tttctcactt tgatgggcat ttgaatcacc cgaggctctt 58380

agtaaactac agattgtgat tcagtggccc tggggttggt tctgagattc agctgatgtt 58440

gctggtctgc agaccatcct ttgagtcata aagttaggac aattacaaga atttagtttg 58500

tcctctgaat gagatgcgaa gtttttagaa agctttaagc agagtgacat gatcttactt 58560

atatttttaa tggattacac tgactgctgc ttggagaatt gattctggtg ggaaagcttg 58620

gaagcaggga gactatttag gaggctactg caaaatttca tacatcaaag tcaagagtgc 58680

atcagtcagg atttgaatgc gggtcagaat taaaacccct aatataaaag catttataat 58740

attagtcccc gatttaaata attacacttt tgtctcttct ccaacagagt ctcaataggt 58800

acagaccaca tccatagctc tcattttaca actcagttaa ttaaaaccta aacgttaatt 58860

tttttagagt atcacagata gcaattagta acaaaagtgg ctcttagata agctgccagt 58920

tatttgaaat caaaataatt atgatgcttg caacagtaac ctgttgacag aaggaactga 58980

gttaaaataa tgaaaaattt tcaaaatact ccatgatcac catccatact tacttcaata 59040

gtcttgaagc atcataacag tcaatggttt ctctgtaatt attaacaata taaaattata 59100

tttaccatga tttcaatgct aaatgttctc atgtgtagtt aagaattgaa aacaggccgg 59160

gcgcggtggc tcacgcctgt aatcccagca ctttgggagg ccgaggcggg tggatcatga 59220

ggtcaggaga tcgagaccat cctggctaac aaggtgaaac cccgtctcta ctaaaaatac 59280

aaaaaattag ccgggcgcgg tggcgggcgc ctgtagtccc agctactcgg gaggctgagg 59340

caggagaatg gcgtgaaccc gggaagcgga gcttgcagtg agccgagatt gcgccactgc 59400

agtccgcagt ccgacctggg cgacagagcg agactccgtc tcaaaaaaaa aaaaaaaaaa 59460

aaaaaaaaaa aaaaaaaaaa gaattgaaaa cagttaacct cattaagtga ttcttaacct 59520

actatggtca aggaaccctt tgataaaatt ataaaacctg taaccctctt ctcttaagca 59580

ttcacataaa aacatacata caaaaatgtt cttacttttt cagtggggtt catgaaccca 59640

ttgaggtgca gattgcacaa aacagattta aggtaacctg tttatcaact tgaatatttc 59700

agtaatgcaa tacatctttt ccttaccatg ctgaataaac agttctctac ttttcttttt 59760

tttttttttt tttttttttt gagatggagt ctcgctctgt cgcccaggct ggagtgcaat 59820

ggcgcgatct tggctcactg caagctccgc ctcccgggtt cacgccattc tcctgcctca 59880

gcctcccaag tagctgggag ttctttacta ttaatatgca tgccaatgca tattcctgtg 59940

attactagcc tttggccaaa tagtgcctga ataaaatgag aatcaagatg atattgagat 60000

gttttcttac cagatacctc ttccccaagg ggtacaaaga gtgtggagaa cgtggcatat 60060

gaaggataat ttgagtgcaa gagtagagcc gggggattgt agattagcat tatcacaggc 60120

tgcccactaa gcaatggctc tttatcccag ttgctatcat aaccttatac acaggggaaa 60180

gggtggaatt agaagtaact tggtgctatg ctagtaagca gtagggaaaa agcagaatct 60240

tatcaggata aatggtacaa aatccatatg tcattttcca ttctccagaa tcagccttgt 60300

aattctacta aatttacaac tctaaataaa gtattctgat ggcaatacat tgttcaatta 60360

tggctctttc tctgactcag aaaaaatcat gattttatca agttgaatac tttttcacct 60420

ggggggaggt gatttatagt cactagtttg ctttaagatg agcctctatg ggtacttttt 60480

acttttattt tctgtcatta attgactggc ccaggtacgt gtaaagcagg agtgtccaaa 60540

ttttgggttc cctgggccac attggaagaa gaagaattgt cttgggccac acataaaata 60600

cactaacaac agttgatgag ctaaaaaaaa ttgcaaaaag aaattcatta tgtttttaga 60660

aagtttatga atttgtgttg ggctgcattc aaggccatct tgggccacat gcaacccaca 60720

gaccgtgggt aggacaagct ttgtgtagag tctatgagcc agatcttctg gctacctctt 60780

cagcaaaatc tgctgttaaa cactggcttg atttggtgaa ttaggttcag agacagctgc 60840

aagctcaacc tggtacccta acgattcgtg tagcatacac ataatattct tagtatgatg 60900

ttaatgagtc tatcatgaag aaaacattcc tcttatgcag gtacaaatat gagagatatt 60960

ctccttcctc caattccact ctcagcatct ctccgtgtct cctagagtca agcagtcaat 61020

taccagtttc agttatggaa aaatcaaaat ccaaaataag taacg 61065

## Claims

1. An *in vitro* method for the prediction of the response of a patient with rheumatoid arthritis (RA) to treatment with tumor necrosis factor inhibitor alpha (anti-TNF alpha agent) selected between infliximab or etanercept that comprises determining, from a sample obtained from the patient, the genotype for single nucleotide polymorphism (SNP) rs3794271, and/or at least one SNP that is in linkage disequilibrium with SNP rs3794271, wherein:
i. the presence of at least one G allele at SNP rs3794271 is indicative of a bad response to treatment while the presence of two alleles other than G at SNP rs3794271 is indicative of a good response to therapy; and/or
ii. the presence of at least one allele correlated with the G allele at SNP rs3794271 for the SNP in linkage disequilibrium is indicative of a bad response to treatment while the presence of two alleles not correlated with the G allele at SNP rs3794271 for the SNP in linkage disequilibrium is indicative of a good response to treatment.

2. The method according to claim 1, where the presence of two G alleles at SNP rs3794271 indicates a predisposition to a bad response to treatment.

3. The method according to any one of claims 1-2, wherein the genotype is determined for SNP rs3794271.

4. The method according to any one of claims 1-3, wherein the obtained sample is selected from the group that comprises plasma, blood, serum and saliva.

5. The method according to any one of claims 1-4, wherein the genotype determination is performed using a DNA analysis technique selected from the group including sequencing, hybridization, Restriction-Fragment Length Polymorphisms, Random Amplification of Polymorphic DNA (RAPD), Polymerase Chain Reaction (PCR) or Amplified Fragment Length Polymorphisms (AFLPD) and a combination thereof.

6. A method to decide or recommend a treatment for a patient with RA that comprises the determination of a SNP rs3794271 genotype and/or a SNP that is in linkage disequilibrium with rs3794271 according to any one of claims 1-5, wherein if the patient has a predisposition to a bad response to treatment, a therapy that excludes infliximab and etanercept is recommended.

7. A method to decide or recommend a treatment for a patient with RA that comprises the determination of a rs3794271 SNP genotype and/or a SNP that is in linkage disequilibrium with rs3794271 according to any one of claims 1-5, wherein if the patient has a predisposition to a good response to treatment, a therapy that includes infliximab or etanercept is recommended.

8. The method according to claim 6, wherein the genotype for SNP rs3794271 is determined according to any one of claims 3-5, and if the patient presents at least one G allele, a therapy that excludes infliximab or etanercept is recommended.

9. The method according to claim 5, wherein the genotype for SNP rs3794271 is determined according to any one of claims 3-5, and if the patient presents two alleles other than G, a therapy that includes infliximab or etanercept is recommended.

10. The method according to any one of claims 1, 2, 4, 5, 6 and 7, wherein the SNP in linkage disequilibrium is located in a locus for predisposition to a bad response to treatment as defined by SEQ ID NO: 1.

11. The method according to claim 10, wherein the SNP in linkage disequilibrium is selected from the group consisting of rs10841585, rs10841586, rs4451779, rs10743388, rs10770689, rs6487129, rs10770691, rs11045376, rs11045378, rs10770695, rs12301364, rs10841593, rs151131008, rs11045385, rs10770701, rs954866, rs3809209, rs7305718, rs74406912, rs11045390, rs11045392, rs2203493, rs137927950, rs2417861, rs3751218, rs959346, rs10770702, rs10743389, rs10770704, rs71939085, rs10743390, rs11392906, rs201855778, rs10770705, rs10770706, rs78690605, rs10505868, rs1473993, rs3838816 and rs10770707.

12. The method according to claim 11, wherein the SNP in linkage disequilibrium is selected from the group consisting of rs10770707, rs1473993, rs10770704, rs10770702, rs959346, rs3751218, rs11045392, rs11045390, rs74406912, rs10770701 and rs954866.

13. Use of SNP rs3794271, and/or a SNP that is in linkage disequilibrium with SNP rs3794271, as a marker of predisposition to the response to treatment with an anti-TNF alpha agent selected between infliximab and etanercept in a patient with RA.

14. Use according to claim 13, wherein the SNP in linkage disequilibrium is located in a locus predisposing to a bad response to treatment as defined by SEO ID NO: 1.

15. Use according to claim 14, wherein the SNP in linkage disequilibrium is selected from the group consisting of rs10841585, rs10841586, rs4451779, rs10743388, rs10770689, rs6487129, rs10770691, rs11045376, rs11045378, rs10770695, rs12301364, rs10841593, rs151131008, rs11045385, rs10770701, rs954866, rs3809209, rs7305718, rs74406912, rs11045390, rs11045392, rs2203493, rs137927950, rs2417861, rs3751218, rs959346, rs10770702, rs10743389, rs10770704, rs71939085, rs10743390, rs11392906, rs201855778, rs10770705, rs10770706, rs78690605, rs10505868, rs1473993, rs3838816 and rs10770707.

16. Use according to claim 15, wherein the SNP in linkage disequilibrium is selected from the group consisting of rs10770707, rs1473993, rs10770704, rs10770702, rs959346, rs3751218, rs11045392, rs11045390, rs74406912, rs10770701 and rs954866.

17. Use according to claim 13, wherein the marker is SNP rs3794271.

18. A kit to predict the response to treatment with an anti-TNF alpha agent selected between infliximab or etanercept in a patient with rheumatoid arthritis which includes means to determine the genotype of SNP rs3794271 and/or at least one SNP that is in linkage disequilibrium with SNP rs3794271, and instructions to perform said determination and for the interpretation of the results, wherein said instructions for the interpretation of the results indicate that the presence of at least one G allele at SNP rs3794271 and/or the presence of an allele correlated with the G allele at SNP rs3794271 for the SNP in linkage disequilibrium is indicative of a bad response to treatment, while the presence of two alleles other than G at SNP rs3794271 and/or the presence of two alleles not correlated with the G allele at SNP rs3794271 for the SNP in linkage disequilibrium is indicative of a good response to treatment.

19. The kit according to claim 18, wherein the SNP in linkage disequilibrium is located in a locus predisposing to a bad response to treatment as defined by SEQ ID NO: 1.

20. The kit according to claim 19, wherein the SNP in linkage disequilibrium is selected from the group consisting of rs10841585, rs10841586, rs4451779, rs10743388, rs10770689, rs6487129, rs10770691, rs11045376, rs11045378, rs10770695, rs12301364, rs10841593, rs151131008, rs11045385, rs10770701, rs954866, rs3809209, rs7305718, rs74406912, rs11045390, rs11045392, rs2203493, rs137927950, rs2417861, rs3751218, rs959346, rs10770702, rs10743389, rs10770704, rs71939085, rs10743390, rs11392906, rs201855778, rs10770705, rs10770706, rs78690605, rs10505868, rs1473993, rs3838816 and rs10770707.

21. The kit according to claim 15, wherein the SNP in linkage disequilibrium is selected from the group consisting of rs10770707, rs1473993, rs10770704, rs10770702, rs959346, rs3751218, rs11045392, rs11045390, rs74406912, rs10770701 and rs954866.

22. The kit according to claim 18, wherein the means are used to determine the genotype for SNP rs3794271 and the instructions indicate that the presence of at least one G allele at SNP rs3794271 is indicative of a bad response to treatment, while the presence of two alleles other than G at SNP rs3794271 is indicative of a good response to treatment.
